# EUROPÄISCHE PATENTSCHRIFT

(11) **EP 2 999 431 B1**
(45) Veröffentlichungstag und Bekanntmachung des Hinweises auf die Patenterteilung: **14.11.2018**
(21) Anmeldenummer: 14716578.1
(22) Anmeldetag: 10.04.2014
(51) Int. Cl.: A61F 2/16

(54) **INJEKTORVORRICHTUNG ZUM EINFÜHREN EINER INTRAOKULARLINSE IN EIN AUGE**
INJECTOR DEVICE FOR INTRODUCING AN INTRAOCULAR LENS INTO AN EYE
DISPOSITIF INJECTEUR POUR L'INTRODUCTION D'UNE LENTILLE INTRAOCULAIRE DANS UN OEIL

(30) Priorität: 21.05.2013 DE 102013105184
(43) Veröffentlichungstag der Anmeldung: 30.03.2016
(73) Patentinhaber: Carl Zeiss Meditec AG, 07745 Jena (DE)
(72) Erfinder: RAQUIN, Vincent, 17000 La Rochelle (FR); PANKIN, Dmitry, 13355 Berlin (DE); RATHERT, Brian, 45659 Recklinghausen (DE)
(74) Vertreter: Hofstetter, Schurack & Partner
(86) Internationale Anmeldenummer: PCT/EP2014/057285
(87) Internationale Veröffentlichungsnummer: WO 2014/187611

(56) Entgegenhaltungen:
- US-A- 5 947 975
- US-A1- 2006 271 063
- US-A1- 2008 077 237

## Beschreibung

Die Erfindung betrifft eine Injektorvorrichtung zum Einführen einer Intraokularlinse in ein Auge, welche eine Injektorspitze mit einem durchgehenden Führungskanal für die Intraokularlinse aufweist, welcher einen hinteren Eingang und einen vorderen Ausgang aufweist.

### Stand der Technik

Intraokularlinsen werden als Implantate in ein Auge eingesetzt und ersetzen die natürliche Linse. Dazu sind Injektorvorrichtungen vorgesehen, welche einen Kolben aufweisen, der in einem Injektorrohr geführt wird. Am vorderen Ende des Injektorrohrs ist ein Aufnahmeraum für die Intraokularlinse gebildet, wobei dieser Aufnahmeraum in einer separaten Kassette ausgebildet sein kann, welche in einen Rahmen des Injektorrohrs einführbar ist. Es kann auch vorgesehen sein, dass der Aufnahmeraum integral in dem Injektorrohr ausgebildet ist. Darüber hinaus ist anschließend an den Aufnahmeraum nach vorne hin eine Injektorspitze ausgebildet, die einen Führungskanal aufweist, in dem die Intraokularlinse nach dem Ausschieben aus dem Aufnahmeraum hindurchgeschoben wird und in einem gefalteten Zustand vorne austritt und in das Auge eingesetzt wird. Die vordere Seite der Spitze ist direkt in das Auge eingeführt.

Bei bekannten Intraokularlinsen tritt mit herkömmlichen bekannten Injektorspitzen und Injektorvorrichtungen das Problem auf, dass sie im Hinblick auf ihre Faltung entweder unkoordiniert sich zusammenrollen, so dass gerade bei asymmetrischen Linsen, die einen optischen Teil mit unterschiedlichen gekrümmten Oberflächen aufweisen, gegebenenfalls das Falten in eine unerwünschte Richtung erfolgt.

Aus der US 2004/0267359 A1 ist eine Injektorspitze für eine Injektorvorrichtung bekannt, welche integriert eine an den hinteren Eingang nach hinten anschließende Kassette aufweist, in der die Intraokularlinse gelagert werden kann. Die Kassette umfasst zwei Deckelklappen, die relativ zueinander um eine Längsachse der Injektorspitze verschwenkbar sind. Die Kassette umfasst zwei rinnenartige Aufnahmen, wobei je eine rinnenartige Aufnahme einem Deckel zugeordnet ist. In diesen rinnenartigen Aufnahmen ist die Intraokularlinse angeordnet, wobei sich beim Schließen der Deckel eine rohrförmige Aufnahme aus den rinnenartigen Aufnahmen bildet, in welcher die Linse vorgefaltet wird, bevor sie in die Injektorspitze eintreten kann. An die jeweiligen rinnenartigen Aufnahmen sind plattenartige Elemente angeformt, die mit den Anlageflächen im geschlossenen Zustand der Deckel aneinanderliegen.

Aus der US 2006/0271063 A1 ist eine Injektorvorrichtung mit einer Injektorspitze bekannt. Überstände, die sich in Richtung der Längsachse des Injektors nach hinten erstrecken, kontaktieren beim Einsetzen eines Moduls mit der Intraokularlinse mit Klappen des Moduls, wodurch sich diese Klappen dann schließen können.

Aus der US 2008/0077237 A1 ist ein System bekannt, mit welchem eine Intraokularlinse verpackt und versendet werden kann.

Darüber hinaus ist aus der US 5,947,975 eine Kassette für eine Intraokularlinse bekannt, bei der ein Grundkörper ausgebildet ist, an den an gegenüberliegenden Seiten in sich starre Deckelklappen angeformt sind, die relativ zum Grundteil verschwenkbar daran angeordnet sind. Die Linse ist in Auflagepodesten dieser Deckelklappen angeordnet und beim Verschließen der Deckelklappen wird sie verformt.

Bei den bekannten Ausführungen ist das Vorfalten der Linse vor deren Eintritt in die Injektorspitze aufgrund der Ausgestaltung der Kassette im Hinblick auf den Ablauf beim Falten eingeschränkt.

### Darstellung der Erfindung

Es ist Aufgabe der vorliegenden Erfindung, eine Injektorvorrichtung zu schaffen, bei welcher das Vorfalten der Intraokularlinse verbessert ist.

Diese Aufgabe wird durch eine Injektorvorrichtung gemäß dem Anspruch 1 gelöst.

Eine erfindungsgemäße Injektorvorrichtung zum Einführen einer Intraokularlinse in ein Auge umfasst eine Injektorspitze mit einem durchgehenden Führungskanal für die Intraokularlinse. Der Führungskanal weist einen hinteren Eingang und einen vorderen Ausgang auf. Dies ist in Richtung der Längsachse der Injektorspitze und somit auch des Führungskanals sowie somit auch der Injektorvorrichtung gesehen. Der hintere Eingang bezeichnet dabei diejenige Stelle, an welche eine Intraokularlinse in die Injektorspitze eintritt, um dann hindurchgeführt zu werden und am vorderen Ausgang dort wieder auszutreten und dann in das Auge eingeführt zu werden.

Die Injektorvorrichtung umfasst zumindest zwei Verschließspitzen, welche zum Verschließen einer die Intraokularlinse aufnehmenden Kassette ausgebildet sind. Die beiden Verschließspitzen erstrecken sich in Richtung der Längsachse der Injektorvorrichtung betrachtet axial weiter nach hinten als der hintere Eingang. Mit der Ausgestaltung bezüglich Anzahl und Form sowie der örtlichen Lage der Verschließspitzen lässt sich das Falten einer Intraokularlinse in der Injektorvorrichtung verbessern. Die Verschließspitzen, welche auch als Zinken oder Kufen oder Finger bezeichnet werden können, ermöglichen ein sehr bedarfsgerechtes und lagegenaues sowie situations- und zeitgenaues Beginnen der Faltung der Intraokularlinse sowie dem nachfolgenden Falten, so dass unerwünschte abrupte Faltvorgänge oder unerwünschte Faltungen in Form und Lage der Intraokularlinse verhindert werden können.

Vorzugsweise ist vorgesehen, dass sich die Verschließspitzen beabstandet und parallel zueinander in Richtung der Längsachse der Injektorvorrichtung erstrecken. Durch eine derartige Anordnung ist bei einer Relativbewegung zwischen den Verschließspitzen und der Kassette, die die Intraokularlinse aufnimmt, die in axialer Richtung durchgeführt wird, ein besonderes gleichmäßiges Verschwenken von Deckelklappen der Kassette ermöglicht. Unerwünschte abrupte Faltvorgänge der Intraokularlinse, die gegebenenfalls mit unerwünschten Kraftbeaufschlagungen sowie Kraftspitzen einhergehen, können dadurch vermieden werden. In dem Zusammenhang kann somit dann auch ein unerwünschtes Verrutschen oder Verdrehen der Linse in der Kassette verhindert werden. Durch die beabstandete Anordnung der Verschließspitzen wird auch ein weiterer Vorteil bezüglich der dann zu erzeugenden Hebelkräfte auf die Deckelklappen erreicht und bei weiterer axialer Verschiebung der Komponenten zueinander somit quasi auch eine seitliche Stütze der Deckelklappen durch die Verschließspitzen ermöglicht. Es kann somit quasi die Kassette seitlich durch die Verschließspitzen gestützt werden, so dass diese multifunktionell gestaltet sind. Neben ihrer Funktion, die Deckelklappen der Kassette bei dieser axialen Relativverschiebung zu schließen, kommt zusätzlich die Führungsfunktion und seitliche Stabilisierung der Kassette durch diese Verschließspitzen hinzu. Gerade durch die Anordnung und die Form der Verschließspitzen wird einerseits auch die exakte axiale Verschiebung der Kassette stabilisiert.

Vorzugsweise ist vorgesehen, dass die Verschließspitzen symmetrisch zueinander zur Längsachse ausgebildet und angeordnet sind. Die oben genannten Vorteile werden dadurch nochmals begünstigt.

Es ist vorgesehen, dass freie hintere Enden der Verschließspitzen verjüngt ausgebildet sind und jeweils eine gebogene Führungsfläche aufweisen. Eine derartige Ausgestaltung ist besonders vorteilhaft, da beim Kontaktieren der Verschließspitzen mit der Kassette auch dann hier keine Barriere überwunden werden muss, die zu einem Stoppen der axialen Verschiebbewegung führt und im Weiteren dann ein erhöhter Kraftaufwand zum Überwinden dieser Barriere erforderlich wäre. Durch diese spezifische Formgebung der Verschließspitzen kann dies verhindert werden und es wird ein ruckfreier und kontinuierlicher Bewegungsablauf zur axialen Verschiebung auch beim Kontaktieren der Verschließspitzen mit der Kassette und auch danach ermöglicht. Besonders begünstigt ist die ruckfreie und gleichmäßige Schwenkbewegung der Deckelklappen durch diese gebogenen Führungsflächen erreicht. Auch hier treten im Übrigen dann die bereits oben genannten Vorteile bezüglich der gewünschten Faltung der Intraokularlinse in der Kassette besonders hervor.

Es ist vorgesehen, dass bei einer Ausführung die Führungsfläche einen steilkurvenartigen hinteren Abschnitt aufweist. Dieser gewundene Abschnitt, der auch quasi einen kurzen Abschnitt einer Helixwindung darstellt, ermöglicht die oben genannten Vorteile bezüglich einer sehr gleichmäßigen Schwenkbewegung der Deckelklappen der Kassette um die Längsachse der Injektorvorrichtung bei auch fortführender axialer Relativverschiebung zwischen den Verschließspitzen und der Kassette.

Vorzugsweise ist vorgesehen, dass dieser steilkurvenartige hintere Abschnitt an dem freien Ende einer Verschließspitze mit einem hinteren Ende beginnt, welches in geradliniger Verbindung zu der Längsachse der Injektorvorrichtung weiter beabstandet ist, als ein vorderes Ende dieses steilkurvenartigen hinteren Abschnitts. Dies bedeutet, dass die Lage des steilkurvenartigen hinteren Abschnitts beginnend von dem hinteren Ende in Richtung zur Längsachse hin krümmt. Eine derartige Ausgestaltung bekräftigt die oben genannten Vorteile nochmals wesentlich. Darüber hinaus ist es bei einer derartigen Ausgestaltung auch erreicht, dass das trichterartige Hinführen der zu verschwenkenden Deckelklappen der Kassette zu einem vorderen Abschnitt der Führungsfläche begünstigt ist. Dieser vordere Abschnitt ist dann vorzugsweise als Schlitz ausgebildet, der zur Führung der dann bereits insbesondere geschlossenen Deckelklappen bei weiterer Axialverschiebung der Kassette relativ zu den Verschließspitzen dient.

Vorzugsweise ist vorgesehen, dass die Neigung der Führungsfläche an dem hinteren Ende und somit eine Tangente an die Führungsfläche am hinteren Ende in einem bevorzugten Winkel zwischen 60° und 100° zu einer Tangente, die am vorderen Ende der Führungsfläche gebildet ist, steht, wobei die beiden Tangenten dabei in einer Ebene betrachtet werden, die senkrecht zur Längsachse der Injektorvorrichtung steht. Ist dann noch die zwischen den beiden Enden liegende Führungsfläche entsprechend kontinuierlich und stufenfrei gebogen und insbesondere dann auch nur in eine Richtung gebogen und somit eine Krümmungsrichtung aufweisend, so ist die steilkurvenartige Führungsfläche an dem hinteren Abschnitt entsprechend weiter konkretisiert.

Zumindest eine Verschließspitze umfasst bei der genannten vorteilhaften Ausführung eine Oberseite, die an den oberen Rand der Führungsfläche angrenzt. Die Oberseite ist vorzugsweise eben ausgebildet. Darüber hinaus umfasst eine Verschließspitze auch noch eine Außenseite, die ebenfalls an die Führungsfläche angrenzt und auch an die Oberseite angrenzt und im Wesentlichen ebenfalls vorzugsweise eben ausgebildet ist. Demgegenüber ist eine Unterseite gebildet, die im Wesentlichen sich in einem vorderen Abschnitt der Verschließspitze erstreckt, wobei diese Unterseite vorzugsweise ebenfalls gewölbt bzw. bogenförmig ausgebildet ist. Die Wölbung ist in dem Zusammenhang ebenfalls vorzugsweise in Richtung zur Oberseite und zur Außenseite hin gebildet und somit vorzugsweise konvex.

Es ist vorgesehen, dass der steilkurvenartige hintere Abschnitt ein dünnes hinteres Ende aufweist, welches sich bis zu einer Mittendicke kontinuierlich verbreitert und dann von der Mittendicke sich kontinuierlich wieder bis zu einem vorderen Ende dieses hinteren Abschnitts verdünnt. Eine derartige Spezifikation beschreibt die Flächengeometrie der Führungsfläche nochmals konkreter. Durch diese Ausgestaltung werden die oben genannten Vorteile bezüglich einer verbesserten Führung und einer kontinuierlicheren Verschwenkung der Deckelklappen mit der erforderlichen Stützfunktion der Verschließspitzen nochmals verbessert.

Andererseits wird gerade durch die Verdünnungen an den Enden und somit den auch verjüngt auslaufenden Flächenenden der Führungsfläche einerseits eine sehr kleinflächige Kontaktierung mit den Außenseiten der Deckelklappen bei der frontseitigen Erstkontaktierung erreicht, so dass hier zunächst keine hohen Reibungen oder mechanischen Verspreizungen auftreten können. Mit der dann sich vergrößernden Führungsfläche werden die mechanische Kontaktierung und das Anlegen zwischen der Führungsfläche und den Deckelklappen vergrößert, so dass das Führungsverhalten und die Stabilisierung verbessert sind.

Vorzugsweise ist vorgesehen, dass der steilkurvenartige hintere Abschnitt in einen parallel zur Längsachse sich erstreckenden vorderen Abschnitt der Führungsfläche mündet. Durch diese Separierung in zumindest zwei Abschnitte der Führungsfläche können die bereits oben genannten unterschiedlichsten Funktionalitäten erreicht werden. Durch gerade diese Anordnung in axialer Richtung werden die jeweils benötigten Funktionen sehr exakt bezüglich der mechanischen Anforderungen und auch bezüglich der zeitlichen Phasen, zu denen sie wesentlich benötigt werden, erreicht, wenn das axiale Verschieben der genannten Komponenten im gewissen zeitlichen Ablauf zueinander und für eine gewisse Zeitdauer erfolgt.

Vorzugsweise ist vorgesehen, dass die Führungsflächen der Verschließspitzen jeweils einen vorderen Abschnitt aufweisen, wobei zumindest zwischen den vorderen Abschnitten ein Führungsschlitz zum Führen in axialer Richtung für Deckelklappen der Kassette ausgebildet ist.

Die diesbezüglich zu erreichenden Vorteile sind bereits oben ausführlich erläutert. Gerade bei dem oben genannten ersten Ausführungsbeispiel der spezifischen Form der hinteren Abschnitte der Verschließspitzen ist die Breite senkrecht zur Längsachse betrachtet dieses Führungsschlitzes kleiner als der Abstand zwischen den hinteren Abschnitten der Führungsfläche. Durch einen derartigen schmalen Führungsschlitz kann eine auch insbesondere passgenaue Führung für die geschlossenen Deckelklappen bei weiterer axialer Verschiebung relativ zu den Verschließspitzen ermöglicht werden, so dass ein ungewolltes wieder teilweise Öffnen der Deckelklappen verhindert ist und darüber hinaus auch eine gesamte Drehung der Kassette um die Längsachse verhindert ist. Die Lage der Intraokularlinse, wie sie in diesem vorgefalteten Zustand in der Kassette vor dem Einführen in den Führungskanal der Injektorspitze erreicht ist, wird somit unverändert und lagegenau aufrecht erhalten. Das weitere Falten in dem Führungskanal der Injektorspitze selbst kann dann wie gewünscht fortgesetzt werden und am Ausgang des Führungskanals wird genau diejenige Endfaltung der Linse erreicht, wie sie dann bestmöglich in das Auge eingeführt werden kann, insbesondere, um eine kleinschnitttaugliche Einführung erreichen zu können.

Vorzugsweise ist vorgesehen, dass sich die Verschließspitzen in einen in einem an die Injektorspitze anschließenden Injektorrohr ausgebildeten, insbesondere rinnenartigen, Aufnahmeraum für die Kassette erstrecken und an einer Innenseite einer vorderen Begrenzungswand des Aufnahmeraums münden. Durch eine derartige Ausgestaltung sind die Verschließspitzen einerseits mechanisch stabil befestigt, andererseits im gewissen Maße versenkt und geschützt angeordnet. Ein unerwünschtes daran Anstoßen mit anderen Komponenten kann dadurch verhindert werden, so dass ein Verbiegen oder Abbrechen der Verschließspitzen verhindert werden kann.

Vorzugsweise ist vorgesehen, dass die Verschließspitzen einstückig mit der Injektorspitze ausgebildet sind. Vorzugsweise kann auch eine einstückige Ausgestaltung der Verschließspitzen mit dem Injektorrohr vorgesehen sein. Insbesondere sind die Verschließspitzen jeweils einstückig aus Kunststoff ausgebildet und bei der integralen Ausgestaltung mit der Injektorspitze und/oder dem Injektorrohr ist dann ebenfalls jeweils ein einstückiger Körper gebildet.

Es ist vorgesehen, dass die Injektorvorrichtung eine Kassette zur Aufnahme der Intraokularlinse aufweist, wobei die Kassette ein Grundteil umfasst, an dem relativ zum Grundteil um die Längsachse der Injektorvorrichtung schwenkbar die Deckelklappen angeordnet sind. Durch diese Flügel bzw. Deckelklappen kann eine besonders einfache und leicht zugängliche Einbringung der Intraokularlinse in die Kassette ermöglicht werden und darüber hinaus dann auch das weitere Vorfalten in der Kassette besonders geeignet erfolgen.

Insbesondere ist die Intraokularlinse dann bereits in der Kassette angeordnet.

Die Kassette kann derart ausgebildet sein, dass sie ein eigenes Modul ist, welches an die Injektorvorrichtung durch einen Nutzer, insbesondere ein medizinisches Personal, mechanisch angebracht werden kann. Es kann jedoch auch vorgesehen sein, dass die Kassette bereits an der Injektorvorrichtung angebracht ist und entsprechend zum medizinischen Personal von dem Hersteller der Injektorvorrichtung geliefert wird.

Die Kassette ist vorzugsweise ebenfalls einstückig ausgebildet, insbesondere aus Kunststoff. In dem Zusammenhang ist es vorteilhaft, wenn das Grundteil mit den Deckelklappen über Schwenkscharniere verbunden sind, die in einer vorteilhaften Ausführung Filmscharniere sein können.

In einer bevorzugten Ausführung ist vorgesehen, dass die Deckelklappen zumindest bereichsweise gebogen bzw. gekrümmt ausgebildet sind. Bei einer bevorzugten Ausführung ist vorgesehen, dass diese Deckelklappen in sich selbst starr und somit ohne Zerstörung unverformbar und unbiegbar ausgebildet sind. Beim Verschwenken um die Längsachse tritt bei einer derartigen Ausgestaltung dann keinerlei Verformung der Deckelklappen in sich selbst auf, insbesondere auch dann nicht, wenn die Deckelklappen an einer anderen Komponente entlang geführt werden bzw. entlangstreifen.

Ein weiterer Aspekt der Erfindung betrifft eine Kassette zur Aufnahme einer Intraokularlinse, wobei die Kassette zur Montage an eine Injektorvorrichtung zum Einführen der Intraokularlinse in ein Auge ausgebildet ist. Die Kassette umfasst ein Grundteil, an dem zwei Deckelklappen relativ dazu bewegbar, insbesondere schwenkbar, angeordnet sind. Die beiden Deckelklappen sind insbesondere in sich selbst zerstörungsfrei verformbar. Dies bedeutet, dass sie ausgehend von einer Grundform senkrecht zur Längsachse der Kassette ohne einen Zerstörung definiert verbiegbar sind, so dass in dem Zusammenhang die gebogene Form einer Deckelklappe verändert werden kann. Diese flexible Verformung der Deckelklappen in sich selbst ist somit dahingehend möglich, dass die grundsätzliche Basisform einer Deckelklappe mit einer bevorzugt zumindest abschnittsweisen gebogenen Form verändert werden kann und somit diese Biegung verkleinert und vergrößert werden kann. Diese Verformung in sich selbst einer Deckelklappe kann zerstörungsfrei reversibel durchführbar und somit vielfach wiederholbar sein. Durch eine derartige Ausgestaltung von in sich selbst verformbaren Deckelklappen der Kassette kann das Falten der Intraokularlinse in der Kassette beim Schließen der Deckelklappen ganz individuell und definiert beeinflusst werden, insbesondere bei einer derartigen Schließbewegung und somit einem Verschwenken der Deckelklappen um die Längsachse der Kassette. Insbesondere ist dabei ein Verformen und somit ein Verbiegen der Deckelklappen in sich selbst bei einer derartigen Verschwenkbewegung der Deckelklappen um die Längsachse zur Längachse hin vorgesehen, so dass in einer Ebene senkrecht zur Längsachse der Kassette betrachtet die Deckelklappen quasi zur Längsachse hin gebogen werden.

Bei einer vorteilhaften Ausführung dieser Kassette weist jede Deckelklappe an einem freien Ende, welches dem Grundteil abgewandt ist, eine hammerkopfförmige Verdickung auf. Durch diese Ausgestaltung ist das geführte Verschwenken der Deckelklappen, insbesondere durch Verschließspitzen einer Injektorvorrichtung, nochmals begünstigt, da gerade dann, wenn die Schwenkbewegung schon weit fortgeschritten ist und die Position der Deckelklappen nahe der Endstellung ist, kein Durchrutschen der Deckelklappen neben den Verschließspitzen möglich ist und somit ein unerwünschtes wieder Öffnen der Deckelklappen verhindert ist. Darüber hinaus wird durch eine derartige Ausgestaltung der Enden der Deckelklappen auch eine mechanisch sehr stabile und selbsthaltende Ausgestaltung erreicht, so dass insbesondere auch im geschlossenen Zustand der Deckelklappen eine mechanisch selbst stabilisierende, im Querschnitt geschlossene Struktur geschaffen ist. Besonders vorteilhaft ist dies gerade bei den in sich verbiegbaren Deckelklappen, die dann im geschlossenen Zustand durch diese robusteren und mechanisch stabilen hammerkopfförmigen Verdickungen gehalten werden und somit auch keinerlei in sich Zusammenfallen der Deckelklappen auftritt, sondern die sehr symmetrische Querschnittform der geschlossenen Kassette aufrecht erhalten ist.

Vorzugsweise ist vorgesehen, dass die hammerkopfförmigen Verdickungen so geformt sind, dass sie beim Verschwenken der Deckelklappen in die geschlossene Stellung zumindest zeitweise auf der Intraokularlinse als Faltstabilisatoren aufsitzen. Dadurch wird die Linse insbesondere an ihren seitlichen Bereichen, insbesondere im optischen Teil, quasi im gewissen Maße umgriffen und das Faltszenario gestützt und unterstützt.

Vorzugsweise wird somit auch ein nach oben Wegrutschen der Intraokularlinse beim Vorfalten in der Kassette verhindert.

Vorzugsweise ist vorgesehen, dass jede Verdickung eine endseitige Anlagefläche aufweist, deren radiale Ausmaße größer sind als eine radiale Dicke der Deckelklappe außerhalb der hammerkopfförmigen bzw. hammerkopfartigen Verdickung.

Vorzugsweise ist vorgesehen, dass die Kassette und die Verschließspitzen in axialer Richtung betrachtet relativ zueinander verschiebbar angeordnet sind. Durch eine derartige Ausgestaltung ist das Vorfalten der Linse in der Kassette sehr begünstigt und definiert durchführbar.

Vorzugsweise weisen Außenseiten der Deckelklappen im Querschnitt betrachtet keinen stufenlosen Konturenverlauf auf, sondern sind gestuft. Vorzugsweise ist an einer Außenseite zumindest eine Erhebung ausgebildet. Insbesondere ist die Erhebung zum Führungseingriff von Verschließspitzen zum automatischen Verschließen der Deckelklappen ausgebildet.

Vorzugsweise ist ein im Querschnitt betrachtet (Ebene senkrecht zur Längsachse) zwischen einer Erhebung und einer endseitigen Verdickung ausgebildeter und im Vergleich dazu (radial betrachtet) dünnerer Klappenabschnitt in sich selbst zerstörungsfrei verbiegbar, insbesondere nur der Abschnitt bzw. Bereich verbiegbar. Durch diese spezifische örtliche Lage und/oder Länge des in sich verbiegbaren Bereichs im Verhältnis zu anderen Teilen einer Deckelklappe werden besondere Vorteile bezüglich verbessertem Faltszenario der Linse in der Kassette erreicht.

Insbesondere ist vorgesehen, dass einen Deckelklappe, insbesondere der Klappenabschnitt, reversibel in sich verformbar ist, so dass die Verformung wiederholt herbeiführbar und wieder rückführbar ist.

Es kann jedoch auch vorgesehen sein, dass die in sich Verformung definiert plastisch und somit nur einmalig erfolgt.

Insbesondere ist die Verformung dann herbeiführbar, wenn sich Endstücke bzw. äußere Enden von zwei Deckelklappen vom Schließen der Deckelklappen berühren aber die Endstücke noch nicht in der Endposition sind, so dass sich beim weiteren Schließvorgang durch die Abstützkräfte der Endstücke aneinander automatisch die gewünschte Verformung der Deckelklappen, insbesondere der Klappenabschnitte, ergibt. Dies erfolgt dann vorzugsweise ohne weitere mechanische Abstützung der Endstücke an anderen Bauteilen.

Vorzugsweise ist vorgesehen, dass die Injektorvorrichtung einen in einem an die Injektorspitze anschließenden Injektorrohr ausgebildeten, insbesondere rinnenartigen, Aufnahmeraum für die Kassette aufweist, der zumindest eine axiale Führungsbahn umfasst, durch welche eine Relativbewegung zwischen dem Führungsrohr und der Kassette axial geführt ist. Dadurch wird die Relativbewegung sehr definiert vorgegeben und gehalten.

Die Führungsbahn ist vorzugsweise ein Schlitz oder eine Nut oder ein Durchbruch an einem Boden des Injektorrohrs. Da das Injektorrohr in sich sehr stabil ist, eignet es sich besonders vorteilhaft als Träger für diese Kassette und ermöglicht in dem Zusammenhang auch, dass die Führungsbahn sehr geradlinig beibehalten bleibt und sich nicht verwindet. Das axiale Verschieben ist dadurch sehr definiert und ruckfrei ermöglicht. Ein Verklemmen oder Verspreizen ist verhindert. Ferner ist dadurch eine integrierte und Bauraum sparende Ausgestaltung der Führungsbahn erreicht.

Besonders vorteilhaft ist es, wenn die Injektorvorrichtung einen Trägerschlitten aufweist und die Kassette mit der Intraokularlinse in dem Trägerschlitten aufgenommen ist. Zum einen wird dadurch die Kassette, die gegebenenfalls etwas filigraner aufgebaut ist und insbesondere dann, wenn die Deckelklappen in sich selbst verbiegbar sind, wird durch den Trägerschlitten ein robustes, mechanisch stabiles Bauteil bereitgestellt, welches einerseits die Aufnahme und andererseits die Bewegung der Kassette vorteilhaft ermöglicht. Die mechanische Wechselwirkung mit anderen Bauteilen der Injektorvorrichtung wird dann über den Trägerschlitten erreicht und insbesondere die axiale Relativbewegung zum Injektorrohr und den Verschließspitzen ist durch diesen Trägerschlitten besonders geführt.

Vorzugsweise ist der Trägerschlitten mit einer Geometrie ausgebildet, die sehr verwindungssteif gebildet ist, so dass die oben genannten Vorteile begünstigt werden. Die Kassette ist vorzugsweise vollständig im Inneren des Trägerschlittens aufgenommen, so dass die Kassette umfangsseitig geschützt ist, andererseits jedoch frontseitig und von hinten zugänglich ist.

Vorzugsweise ist vorgesehen, dass der Trägerschlitten rohrartig ausgebildet ist und die Kassette, insbesondere positionsfixiert, im Inneren des Trägerschlittens angeordnet ist. Dadurch können die oben genannten Vorteile im besonderen Maße erreicht werden. Vorzugsweise ist der Trägerschlitten in dem Aufnahmeraum der Injektorvorrichtung angeordnet, so dass er auch hier in gewissem Maße eingebettet und von unten und seitlich gestützt positioniert werden kann.

Vorzugsweise ist vorgesehen, dass eine Relativbewegung zwischen dem Injektorrohr und der Kassette axial geführt ist. Der Trägerschlitten ist dazu entsprechend mit der Injektorvorrichtung, insbesondere dem Injektorrohr, gekoppelt.

Bei einer bevorzugten Ausführung ist vorgesehen, dass der Trägerschlitten in seiner unverschobenen Grundposition im Aufnahmeraum nach hinten beabstandet zu den Verschließspitzen positioniert ist. Der Aufnahmeraum ist in dem Zusammenhang vorteilhafterweise so dimensioniert, dass in Längsrichtung der Injektorvorrichtung betrachtet die Verschließspitzen und dieser Trägerschlitten quasi in Reihe zueinander angeordnet werden können. Einerseits kann somit eine sehr einfache und nutzerfreundliche Beladung des Aufnahmeraums mit dem Trägerschlitten ermöglicht werden, andererseits ist die Zugänglichkeit sowohl zu den Verschließspitzen als auch zu dem Trägerschlitten uneingeschränkt möglich. Darüber hinaus ist gerade durch eine derartige Dimensionierung des Aufnahmeraums und dem gewünschten Szenario der relativen axialen Verschiebung zwischen den Verschließspitzen und diesem Trägerschlitten mit der Kassette maximal Rechnung getragen und das durch die spezifische Formgebung der Verschließspitzen ganz definierte Bewegungsszenario der Deckelklappen beim axialen Verschieben der Komponenten zueinander kann damit voll zur Geltung kommen.

Vorzugsweise ist vorgesehen, dass der Trägerschlitten ein Griffelement aufweist, welches durch einen Nutzer greifbar ist, wobei der Trägerschlitten mit der Kassette nutzergeführt relativ zu den anderen Komponenten der Injektorvorrichtung axial zum vorderen Ende des Aufnahmeraums verschiebbar ist. Bei einer derartigen Ausgestaltung ist das Injektorrohr ortsfest mit einem Kolbenrohr der Injektorvorrichtung verbunden, wobei das Kolbenrohr in den Ausschiebekolben, mittels welchem die Intraokularlinse aus der Kassette in die Injektorspitze, insbesondere den Führungskanal, eingeschoben wird. Bei dieser Ausgestaltung ist somit eine axiale Relativverschiebung zwischen den Komponenten dadurch erreicht, dass lediglich der Trägerschlitten mit der Kassette bewegt und axial verschoben wird.

Bei einer alternativen Ausgestaltung ist vorgesehen, dass der Trägerschlitten positionsfixiert an einem Kolbenrohr der Injektorvorrichtung angeordnet ist, und ein den Aufnahmeraum aufweisendes Injektorrohr der Injektorvorrichtung, an dem die Injektorspitze angeordnet ist, relativ zum Kolbenrohr mit der Kassette axial verschiebbar ist. Bei dieser Ausgestaltung wird somit nicht der Trägerschlitten mit der Kassette aktiv verschoben, sondern durch einen Nutzer das Injektorrohr quasi relativ zum Kolbenrohr verschoben.

Vorzugsweise ist vorgesehen, dass der Trägerschlitten an seiner Unterseite Führungskufen aufweist, die in eine Führungsbahn im Injektorrohr eingreifen. Durch eine derartige Ausgestaltung kann eine sehr einfache mechanische Kopplung zwischen dem Trägerschlitten und dem Injektorrohr ermöglicht werden und die axiale Bewegungsführung sehr einfach erfolgen. Darüber hinaus ist eine unerwünschte Bewegung in einer Richtung seitlich oder schräg zur Längsachse verhindert.

Bevorzugt sind die Führungskufen als flexible Rastelemente ausgebildet. Diese Rastelemente, die auch als Schnappelemente ausgebildet sein können, ermöglichen eine einfache und sichere mechanische Kopplung der Komponenten und hintergreifen die Führungsbahn. In dem Zusammenhang ist es vorteilhaft, wenn die Führungsbahn ein Durchbruch bzw. ein Schlitz ist, so dass diese Führungskufen in Form der Rastelemente sich durch den Führungsschlitz hindurch erstrecken und außenseitig quasi dann den Rand des Schlitzes umgreifend zum Liegen kommen. Die oben genannten Vorteile werden dadurch besonders begünstigt.

Vorzugsweise ist vorgesehen, dass zwischen einer Innenwand des Trägerschlittens und Außenseiten von schwenkbaren Deckelklappen der Kassette ein Eingriffsraum für die Verschließspitzen ausgebildet ist. Durch diese Ausgestaltung ist einerseits eine ausreichende Bewegung der Deckelklappen zu deren Verschwenkung ermöglicht und andererseits dennoch eine geeignete Führung dieser Verschwenkung erreicht.

Es ist vorgesehen, dass die Verschließspitzen und die Kassette so angeordnet sind, dass bei einer axialen Relativverschiebung zwischen der Kassette und den Verschließspitzen die Verschließspitzen Außenseiten von geöffneten Deckelklappen kontaktieren und bei einem weiteren axialen Verschieben zwischen der Kassette und den Verschließspitzen relativ zueinander die Deckelklappen automatisch durch die Verschließspitzen um die Längsachse der Injektorvorrichtung verschwenkbar und schließbar sind. Die dabei erreichten Vorteile sind bereits oben umfänglich erläutert.

Vorzugsweise ist vorgesehen, dass eine obere Deckenseite einer Innenwand des Trägerschlittens gewölbt, insbesondere kuppelartig gebildet, ist und ein Radius der Wölbung zumindest bereichsweise größer als ein Radius der Wölbung der Deckelklappen ist. Dadurch kann das Schließen der Deckelklappen ungehindert und in gewünschter Weise erfolgen.

Insbesondere ist vorgesehen, dass die Deckelklappen beim Verschwenken um die Längsachse der Injektorvorrichtung zumindest phasenweise bzw. zeitweise während des Verschwenkvorgangs an der Deckenseite anliegen und dadurch in sich verbiegbar sind. Dies kann gerade bei den Ausgestaltungen, bei denen die Deckelklappen in sich selbst verformbar sind, vorgesehen sein. Dieses in sich Verbiegen beim Schließen wird somit entsprechend definiert durchgeführt. Dieses definierte Durchführen erfolgt somit sowohl im Hinblick auf den Grad der Verbiegung als auch im Hinblick auf den Zeitpunkt, zu welchem die Verbiegung während der Verschwenkung der Deckelklappen erfolgen soll. Das im Hinblick auf den Grad und/oder den Zeitpunkt der Verformung hervorgerufene Szenario wird dadurch automatisch durchgeführt.

Vorzugsweise ist vorgesehen, dass hammerkopfförmige bzw. hammerkopfartige Verdickungen an freien Enden der Deckelklappen beim Verschwenken durch das in sich
Verbiegen der Deckelklappen, insbesondere von bogenförmigen Klappenabschnitten, erst mit äußeren Enden der Anlageflächen der hammerkopfartigen Verdickungen kontaktiert sind. Es wird somit zunächst eine linienförmige oder sehr kleinflächige mechanische Kontaktierung zwischen den Verdickungen erreicht. Dies ist besonders vorteilhaft, um beim weiteren Schließvorgang ein definiertes Bewegungsszenario der in sich verbogenen Deckelklappen hervorzurufen, so dass diese wiederum ihre unverformte Grundform einnehmen und bei diesem Vorgang dann auch das definierte Zuendeführen der Vorfaltung der Intraokularlinse in der Kassette gezielt erfolgen kann.

Vorzugsweise sind die Deckelklappen und die Verdickungen so ausgebildet, dass bei einem weiteren Verschwenken um die Längsachse in die geschlossene Endlage der Deckelklappen die Anlageflächen dieser hammerkopfartigen Verdickungen sich aneinander anlehnen und dadurch der in sich verbogene Zustand der Deckelklappen in die Grundstellung automatisch zurückführbar ist. Die Grundstellung ist in dem Zusammenhang mit bogenförmigen Klappenabschnitten versehen, die dann jedoch weniger stark gebogen sind als im in sich verbogenen Zustand.

Bei einer bevorzugten Ausführung der Kassette ist vorgesehen, dass sie neben den Deckelklappen einen nach oben schwenkbaren Sicherungsdeckel für die Linse aufweist. Der Sicherungsdeckel ist somit ein zusätzliches weiteres Bauteil neben den Deckelklappen. Ein nach oben Herausfallen oder ein unerwünschtes nach vorne Rutschen in Richtung der Längsachse ist dadurch verhindert.

Vorzugsweise ist der Sicherungsdeckel relativ verschwenkar gegenüber dem Grundteil der Kassette. Insbesondere ist der Sicherungsdeckel über ein Filmscharnier mit dem Grundkörper bzw. dem Grundteil der Kassette verbunden und relativ dazu nach oben und unten verschwenkbar.

Vorzugsweise weist der Sicherungsdeckel einen nach vorne sich erstreckenden hakenförmigen Sicherungsbügel auf. Die oben genannten Vorteile sind dadurch besonders erreicht und die Deckelklappen im Hinblick auf ihre Formgebung und Lagepositionierung im geöffneten Zustand nicht eingeschränkt.

Vorzugsweise ist vorgesehen, dass der Sicherungsdeckel im geöffneten Zustand der Deckelklappen geschlossen ist.

In einer besonders bevorzugten Ausführung ist vorgesehen, dass der Sicherungsdeckel Anhebeelemente, insbesondere Anhebeflanken, aufweist, die beim Schließen der Deckelklappen und somit bei deren Verschwenken um die Längsachse der Kassette, von diesen kontaktiert sind und bei weiterem Schließen der Deckelklappen ein automatisches Öffnen des Sicherungsdeckels durchführbar ist.

Des Weiteren betrifft die Erfindung ein Verfahren zum Falten einer Intraokularlinse in einer Injektorvorrichtung zum Einführen einer Intraokularlinse in ein Auge, welche eine Injektorspritze mit einem durchgehenden Führungskanal für die Intraokularlinse aufweist, welche einen hinteren Eingang und einen vorderen Ausgang aufweist. Zumindest zwei Verschließspitzen der Injektorvorrichtung, welche sich in Richtung der Längsachse der Injektorvorrichtung axial weiter nach hinten erstrecken als der hintere Eingang, verschwenken bei einer axialen Relativverschiebung zwischen den Verschließspitzen und einer Kassette, in welche die Intraokularlinse angeordnet wird, die geöffneten Deckelklappen der Kassette um die Längsachse, und durch das Verschwenken wird die Intraokularlinse in der Kassette vorgefaltet. Die dadurch erreichbaren Vorteile sind bereits oben genannt.

Vorzugsweise wird beim Verschieben der Kassette in axialer Richtung relativ zu den Verschließspitzen ein Eingreifen der Verschließspitzen zwischen einen Trägerschlitten und der Kassette, die in dem Trägerschlitten aufgenommen ist, erreicht. Die Verschließspitzen legen sich mit Führungsflächen an Außenseiten der Deckelklappen an und durch die Formgebung der Führungsflächen wird bei einem weiteren ausschließlich axialen Verschieben zwischen den Verschließspitzen und der Kassette eine Überführung der Deckelklappen in ihre geschlossene Endlage durchgeführt. Allein durch das ausschließlich axiale Relativverschieben zwischen den Verschließspitzen und der Kassette werden die Deckelklappen der Kassette automatisch um die Längsachse verschwenkt und geschlossen. Insbesondere wird ein Weiterführen der geschlossenen Deckelklappen in einem Führungsschlitz zwischen den Verschließspitzen durchgeführt. Dies erfolgt vorzugsweise so lange, bis eine Endposition zwischen der Kassette und einem Injektorrohr der Injektorvorrichtung erreicht ist und dann anschließend die Intraokularlinse aus der Kassette in die Injektorspitze bzw. deren Führungskanal eingeschoben wird.

Weitere vorteilhafte Ausführungen des erfindungsgemäßen Verfahrens werden durch vorteilhafte Ausführungen der Injektorvorrichtung bzw. der Kassette gebildet. Die hierfür bei der Injektorvorrichtung und der Kassette genannten gegenständlichen Merkmale dienen einzeln oder in Kombination zur Realisierung von entsprechenden Verfahrensschritten.

Bezüglich den Begriffen "hinteres" oder "vorderes" wird dies in Richtung der Längsachse und mit Blick auf die Injektorspitze gesehen zueinander definiert.

Des Weiteren betrifft die Erfindung eine Verpackungs- und Transporteinrichtung für eine Intraokularlinse. Die Einrichtung umfasst einen Transportbehälter und eine erfindungsgemäße Injektorvorrichtung oder eine vorteilhafte Ausgestaltung davon. Die Injektorvorrichtung ist in den Transportbehälter einbringbar und insbesondere im fertig gestellten Zustand der Verpackungs- und Transporteinrichtung ist die Injektorvorrichtung in dem Transportbehälter positionsstabil angeordnet. In diesem Endzustand der Verpackungs- und Transporteinrichtung ist eine Intraokularlinse in der Injektorvorrichtung angeordnet. Bei dieser Verpackungs- und Transporteinrichtung für eine Intraokularlinse kommen die bereits oben erwähnten Vorteile deutlich zum Tragen. Neben einer verbesserten Handhabbarkeit kann die Beschädigung der Intraokularlinse reduziert werden. Darüber hinaus sind keine zusätzlichen Hilfswerkzeuge, wie beispielsweise eine Pinzette oder dergleichen, erforderlich, um die Intraokularlinse aus dem Transportbehälter bei erforderlicher Verwendung bei einem chirurgischen Eingriff zu entnehmen.

Vorzugsweise ist in dem Transportbehälter eine sterile Flüssigkeit enthalten, in welche die Linse eingetaucht angeordnet ist. Nach dem Verpacken und somit auch beim Transport ist die Linse von dieser sterilen Flüssigkeit umgeben, so dass auch hier unerwünschte Kontaminierungen vermieden werden können. Insbesondere ist der Transportbehälter an einer Oberseite mit einer Abdeckung verschlossen, so dass auch hier keine Verunreinigungen in den Aufnahmeraum des Transportbehälters gelangen können. Darüber hinaus ist die Versendung diesbezüglich ohne Flüssigkeitsverlust möglich.

Vorzugsweise ist eine sterile und keimfreie Verschließung des Transportbehälters durch die Abdeckung gewährleistet. So kann hier eine Anklebung der Abdeckung oder eine thermoverschweißte Verbindung vorgesehen sein. Der Transportbehälter ist somit zur Lagerung und zum Transport vollständig geschlossen. Die Abdeckung kann zumindest bereichsweise durchsichtig ausgebildet sein, so dass die Haltevorrichtung und auch die Intraokularlinse in dem Transportbehälter eingesehen werden können. Auf der Abdeckung können Informationen über die Intraokularlinse charakterisierende Parameter angegeben sein.

Insgesamt wird durch die Verpackungs- und Transporteinrichtung und die Injektorvorrichtung ein Gesamtsystem geschaffen, welches einen hochfunktionellen Ablauf von der Herstellung und Verpackung der Linse bis hin zum Einsetzen der Linse ins Auge gewährleistet. Dieses ist insbesondere im Hinblick auf die Einhaltung der Sterilisierungsvorgänge und der keimfreien Behandlung der Linse als auch im Hinblick auf benutzerfreundliche Handhabbarkeit und abgestimmte Abläufe bei der Verwendung der Linse verbessert. Wird quasi eine derartige Linse bei einem chirurgischen Eingriff verwendet und ist die erfindungsgemäße Verpackungs- und Transporteinrichtung der Intraokularlinse oder eine vorteilhafte Ausgestaltung davon dem medizinischen Personal bereits übersandt, so muss nur noch die Verpackung geöffnet werden indem die Abdeckung vom Transportbehälter abgenommen wird. Das medizinische Personal muss nur noch die Injektorvorrichtung aus dem Transportbehälter entfernen und die erläuterten Initialbewegungen durchführen, um die weitere automatische Beladung der Injektorspitze mit der Linse erreichen zu können.

In dem zumindest eine der stirnseitigen Öffnungen des Aufnahmebehälters durch eine sehr dünne Folie verschlossen ist, kann ermöglicht werden, dass die Intraokularlinse bereits direkt in die Kassette eingebracht wird und die Kassette somit multifunktionell ausgebildet ist. Dies, da sie somit auch die integrale Funktion des Transportbehälters aufweist. Die erfindungsgemäße Kassette ist somit eine Komponente, die zum einen den Transportbehälter selbst bildet, zum anderen auch genau das Bauteil bildet, welches mit der Injektorvorrichtung verbunden ist oder verbindbar ist und welches es dann im verbundenen Zustand mit der Injektorvorrichtung ermöglicht, dass die darin befindliche Intraokularlinse aus der Kassette ausgeschoben werden kann. Durch die Folie wird einerseits eine mechanisch stabile Verschließung ermöglicht, die andererseits auch die sterile Aufbewahrung der Intraokularlinse in der Kassette ermöglicht.

Vorzugsweise ist im Inneren des Aufnahmebehälters eine ausgeglichene Salzlösung oder reines Wasser angeordnet. Die darin sich befindliche Intraokularlinse wird dadurch steril aufbewahrt.

Durch die Anbringung einer Folie an zumindest einer der Stirnseiten kann diese Folie auch einfach und aufwandsarm bearbeitet werden, um dann im Nachfolgenden die Intraokularlinse aus dem Aufnahmebehälter ausbringen zu können. Dies insbesondere dann, wenn diese Intraokularlinse durch einen Kolben der Injektorvorrichtung aus dieser Kassette ausgeschoben werden soll.

Insbesondere wird somit nach Fertigstellung der Kassette die Intraokularlinse in den Aufnahmebehälter eingebracht und die ausgeglichene Salzlösung eingebracht. Danach wird zumindest eine der Öffnungen mit der Folie verschlossen. Die so bereitgestellte Kassette umfasst somit einen hermetisch abgeschlossenen Aufnahmebehälter, in dem bereits die Intraokularlinse steril aufbewahrt und integriert ist. Eine derartig ausgebildete Kassette kann dann im Weiteren bereits in eine Injektorvorrichtung integriert werden, sodass bereits die komplette Injektorvorrichtung bereitgestellt und im Nachgang dann ausgeliefert und gegebenenfalls zu einem Operateur angeliefert werden kann. Bei einer derartigen Ausgestaltung ist somit der Arbeitsaufwand und insbesondere der Montageaufwand minimiert.

Besonders vorteilhaft ist es, wenn eine Folie eine koextrudierte Folie ist. Für eine derartige spezifische Folie ist es in besonders hervorzuhebender Weise möglich, dass einerseits eine hermetisch dichte Versiegelung der Öffnung mit der Folie gewährleistet werden kann. Andererseits ist dadurch erreicht, dass auch eine besonders sterile Barriere durch die Folie erzeugt wird, sodass keine unerwünschten Verunreinigungen ins Innere des Aufnahmebehälters gelangen können. Darüber hinaus gewährleistet diese koextrudierte Folie noch die gewünschte Flexibilität im Hinblick auf das nachträgliche Entfernen oder Durchstoßen, wenn die Intraokularlinse aus der Kassette ausgeschoben werden soll.

Vorteilhaft ist es, dass die koextrudierte Folie eine Aluminiumschicht aufweist. Aluminium ist hierbei ein besonders hervorzuhebendes Material, um die sterile Barriere der Folie erreichen zu können.

In vorteilhafter Weise ist vorgesehen, dass die koextrudierte Folie Propylen aufweist, insbesondere eine Polypropylenschicht umfasst. Insbesondere ist diese Materialkomponente zum hermetischen Versiegeln und Anbringen an dem Aufnahmebehälter geeignet. Das Polypropylenmaterial kann dabei besonders dichtend mit dem Material in dem Aufnahmebehälter verbunden werden.

Vorzugsweise ist die koextrudierte Folie dahingehend ausgebildet, dass sie eine Polypropylenlage aufweist, auf der eine Aluminiumlage ausgebildet ist. Die Aluminiumlage ist dann vorzugsweise noch mit einem Lack oder sonstiger Beschichtung bedeckt.

Vorzugsweise ist vorgesehen, dass eine Folie zweilagig an einer Öffnung ausgebildet ist. Dies ist insbesondere dahingehend konzipiert, dass die dem Aufnahmebehälter näher gelegene Lage damit fest verbunden ist, sodass die Versiegelung und sterile Barriere gewährleistet ist. Die zweite, dem Aufnahmebehälter abgewandte Lage ist insbesondere dahin konzipiert, dass sie im Überlagerungsbereich mit der ersten Lage insbesondere nicht fest mit der ersten Lage verbunden ist. Es ist somit eine gewisse Relativbewegbarkeit zu der ersten Lage gewährleistet.

Insbesondere kann die Kassette ein Empfangs-Container zum Speichern einer hydrophilen Linse in einem Wassermedium (reines oder salziges Wasser) sein.

Vorzugsweise ist vorgesehen, dass die Kassette dampfsterilisiert ist und die Injektorspitze und die Injektorröhre mit Ethylenoxid sterilisiert sind.

Weitere Merkmale der Erfindung ergeben sich aus den Ansprüchen, den Figuren und der Figurenbeschreibung. Die vorstehend in der Beschreibung genannten Merkmale und Merkmalskombinationen sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen oder in Alleinstellung verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind.

### Kurze Beschreibung der Zeichnungen

Ausführungsbeispiele der Erfindung werden nachfolgend anhand schematischer Zeichnungen näher erläutert. Es zeigen:
- Fig. 1: eine perspektivische Darstellung eines Ausführungsbeispiels einer erfindungsgemäßen Injektorvorrichtung;
- Fig. 2: eine Teildarstellung der Injektorvorrichtung gemäß Fig. 1 in perspektivischer Schnittansicht;
- Fig. 3: die Injektorvorrichtung gemäß Fig. 1 in einem von Fig. 1 unterschiedlichen Betriebszustand;
- Fig. 4: eine Teildarstellung der Injektorvorrichtung gemäß Fig. 3 in perspektivischer Schnittansicht;
- Fig. 5: eine perspektivische Darstellung eines Ausführungsbeispiels einer erfindungsgemäßen Kassette zur Aufnahme einer Intraokularlinse mit einem zusätzlichen Trägerschlitten;
- Fig. 6: eine weitere perspektivische Darstellung der Vorrichtung gemäß Fig. 5;
- Fig. 7: eine perspektivische Schnittansicht der Vorrichtung gemäß Fig. 5 und Fig. 6;
- Fig. 8: eine perspektivische Darstellung der Vorrichtung gemäß Fig. 5 bis Fig. 7 mit geschlossenen Deckelklappen der Kassette;
- Fig. 9: eine Teildarstellung der Injektorvorrichtung gemäß Fig. 1 in vergrößerter Ansicht;
- Fig. 10: eine perspektivische Darstellung eines Teilausschnitts der Injektorvorrichtung gemäß Fig. 1 bis 4 in einem weiteren Betriebszwischenzustand;
- Fig. 11: eine perspektivische Darstellung eines Ausführungsbeispiels einer erfindungsgemäßen Kassette im geöffneten Zustand der Deckelklappen;
- Fig. 12: die Kassette gemäß Fig. 11 mit geschlossenen Deckelklappen;
- Fig. 13a-13c: Schnittdarstellungen von Teilansichten von verschiedenen Ausführungsbeispielen von Deckelklappen der Kassette gemäß Fig. 11 und Fig. 12 mit teilweise gezeigter Intraokularlinse;
- Fig. 14: eine Schnittdarstellung der Kassette gemäß Fig. 11 und 12 mit aufgenommener Intraokularlinse im geöffneten Zustand der Deckelklappen;
- Fig. 15: eine Schnittdarstellung der Anordnung gemäß Fig. 14 mit in einem ersten Betriebszwischenzustand mit teilweise verschwenkten Deckelklappen;
- Fig. 16: eine Schnittdarstellung der Anordnung gemäß Fig. 14 und Fig. 15 mit in einem weiteren Betriebszwischenzustand weiter verschwenkten Deckelklappen;
- Fig. 17: eine Schnittansicht der Anordnung gemäß Fig. 14 bis Fig. 16 mit geschlossenen Deckelklappen;
- Fig. 18: eine Schnittdarstellung der Injektorvorrichtung gemäß Fig. 1 bis 3 mit der Kassette mit geschlossenen Deckelklappen gemäß der Darstellung in Fig. 17;
- Fig. 19: eine perspektivische Darstellung eines weiteren Ausführungsbeispiels einer erfindungsgemäßen Injektorvorrichtung;
- Fig. 20: eine perspektivische Darstellung einer Verpackungs- und Transportvorrichtung mit einer Injektorvorrichtung gemäß Fig. 19;
- Fig. 21: eine Draufsicht auf einen Teilausschnitt der Injektorvorrichtung gemäß Fig. 19 mit von oben gezeigten Verschließspitzen;
- Fig. 22: eine perspektivische Darstellung der Komponenten in Fig. 21;
- Fig. 23: eine Seitenansicht von Teilkomponenten der Darstellung in Fig. 21 und Fig. 22;
- Fig. 24: eine Draufsicht auf die Darstellung in Fig. 23;
- Fig. 25: eine weitere perspektivische Darstellung der Komponenten gemäß Fig. 21 und 22;
- Fig. 26: eine perspektivische Schnittansicht der Darstellung in Fig. 25;
- Fig. 27: eine perspektivische Darstellung der Komponenten, wie sie in Fig. 23 und Fig. 24 gezeigt sind;
- Fig. 28: eine perspektivische Darstellung eines Ausführungsbeispiels einer Vorrichtung umfassend einen Trägerschlitten und eine Kassette für eine Intraokularlinse;
- Fig. 29: eine weitere perspektivische Darstellung der Vorrichtung gemäß Fig. 28 ohne endseitige Abdeckelemente;
- Fig. 30: eine perspektivische Darstellung der Vorrichtung gemäß Fig. 29 im in einem Injektorrohr der Injektorvorrichtung aufgenommenen Zustand;
- Fig. 31: eine Frontansicht der Darstellung in Fig. 30;
- Fig. 32: eine Frontansicht eines Ausführungsbeispiels einer erfindungsgemäßen Kassette zur Aufnahme einer Intraokularlinse;
- Fig. 33: eine perspektivische Darstellung der Kassette gemäß Fig. 32 mit teilweise angehobenem Schwenkdeckel und teilweise geschlossenen Deckelklappen;
- Fig. 34: eine Schnittdarstellung der Ausführung in Fig. 33;
- Fig. 35: eine Schnittdarstellung der Injektorvorrichtung gemäß Fig. 19 in einem ersten Betriebszustand;
- Fig. 36: eine Schnittdarstellung der Injektorvorrichtung gemäß Fig. 35 in einem zweiten Betriebszustand;
- Fig. 37: eine Schnittdarstellung der Injektorvorrichtung gemäß Fig. 35 und Fig. 36 in einem weiteren Betriebszustand;
- Fig. 38: eine perspektivische Darstellung der Injektorvorrichtung gemäß Fig. 35 bis Fig. 37 in einem weiteren Betriebszustand;
- Fig. 39: eine perspektivische Schnittdarstellung der Injektorvorrichtung gemäß Fig. 35 bis Fig. 38 in einem weiteren Betriebszustand;
- Fig. 40: eine Darstellung eines Teilausschnitts der Injektorvorrichtung gemäß Fig. 35;
- Fig. 41: eine vergrößerte Teildarstellung der Injektorvorrichtung gemäß Fig. 36;
- Fig. 42: eine vergrößerte Darstellung der Injektorvorrichtung gemäß Fig. 37;
- Fig. 43: eine vergrößerte Darstellung eines Teilausschnitts der Injektorvorrichtung gemäß Fig. 38;
- Fig. 44: eine vergrößerte Teildarstellung der Injektorvorrichtung gemäß Fig. 39;
- Fig. 45: eine vergrößerte Darstellung eines Teilausschnitts der Injektorvorrichtung gemäß Fig. 35 bis 39 in einem weiteren Betriebszwischenzustand zwischen den Betriebszuständen gemäß Fig. 36 und 37; und
- Fig. 46: eine vergrößerte Darstellung der Ansicht in Fig. 45.

### Bevorzugte Ausführung der Erfindung

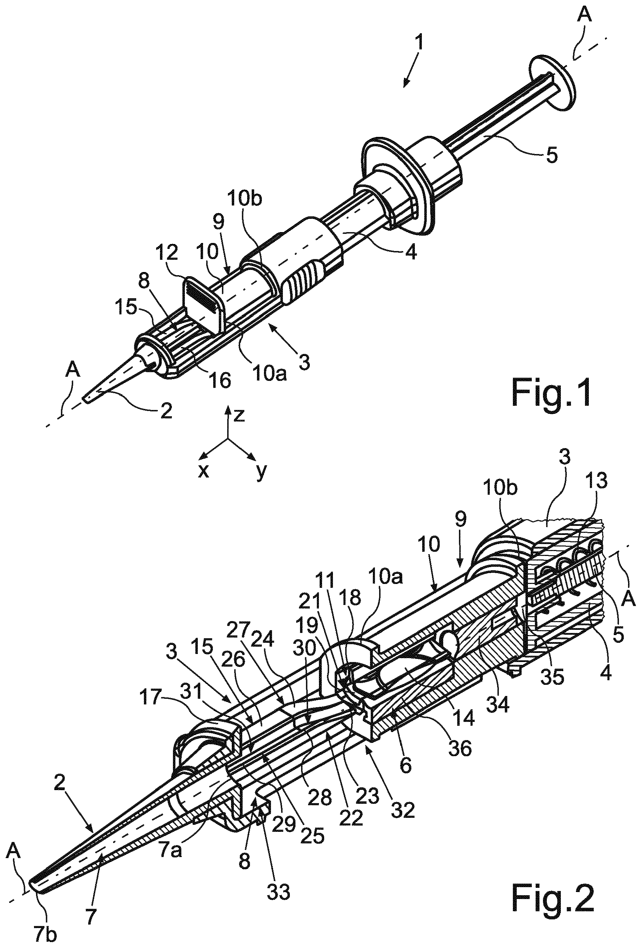

In Fig. 1 ist in einer perspektivischen Darstellung ein Ausführungsbeispiel einer Injektorvorrichtung 1 gezeigt, die zum Einführen einer Intraokularlinse in ein Auge ausgebildet ist. Die Injektorvorrichtung 1 umfasst in der gezeigten Ausführung eine Injektorspitze 2, die mit einem Injektorrohr 3 verbunden ist. Das Injektorrohr 3 ist in Richtung einer Längsachse A der Injektorvorrichtung 1 relativ gegenüber einem Kolbenrohr 4 verschiebbar. Das Kolbenrohr 4 ist zur Aufnahme eines Kolbens 5 vorgesehen, welcher in axialer Richtung verschiebbar ist. Mit dem Kolben 5 wird die Intraokularlinse aus einer Kassette 6 (Fig. 2) ausgeschoben und in einen Führungskanal 7 (Fig. 2) der Injektorspitze 2 eingeschoben.

Wie in Fig. 1 zu erkennen ist, weist das Injektorrohr 3 einen nach oben offenen Aufnahmeraum 8 auf, der zur Aufnahme einer Vorrichtung 9 ausgebildet ist. Die Vorrichtung 9 umfasst die Kassette 6 und einen Trägerschlitten 10. Der Trägerschlitten 10 ist in der gezeigten Ausführung als einstückiges rohrartiges Bauteil gebildet. Im Inneren 11 (Fig. 2) ist die Kassette 6 ortsfest zum Trägerschlitten 10 positioniert.

Der Aufnahmeraum 8 weist eine axiale Länge (x-Richtung) auf, die größer ist als eine Länge der Vorrichtung 9 und somit auch größer ist als die Länge des Trägerschlittens 10. Wie aus den Darstellungen insbesondere in Fig. 1 und Fig. 2 zu entnehmen ist, ist die Kassette 6 vollständig im Inneren 11 aufgenommen und erstreckt sich in axialer Richtung nicht über die Ausmaße des Trägerschlittens 10 hinweg.

In der gezeigten Ausführung in Fig. 1 ist der Trägerschlitten 10 an einem vorderen Ende 10a mit einem Abdeckelement verschlossen. Vorstehend und nachfolgend beziehen sich die Angaben zu "vorderen" und "hinteren" Bauteilen auf deren Lage in Richtung der Längsachse A mit Blick zur Spitze 2 der Vorrichtung 1. Das Abdeckelement 12 kann plattenartig und in sich starr ausgebildet sein. Es kann jedoch auch eine Folie sein, beispielsweise eine koextrudierte Folie. Es kann jedoch auch eine Folie sein, an der ein starres Element zum Greifen angeordnet ist, mit welchem die Folie dann abgezogen werden kann.

An einem hinteren Ende 10b ist der Trägerschlitten 10 ortsfest mit dem Kolbenrohr 4 verbunden. Wie dazu insbesondere in Fig. 2 zu erkennen ist, ist das Kolbenrohr 4 innen hohl und der Kolben 5 durch eine Feder 13 vorgespannt axial verschiebbar.

Die Injektorspitze 2 umfasst den bereits angesprochenen Führungskanal 7, der einen hinteren Eingang 7a und einen vorderen Ausgang 7b aufweist. Der hintere Eingang 7a ist dem Aufnahmeraum 8 zugewandt angeordnet und die aus der Kassette 6 ausgeschobene Intraokularlinse 14 (Fig. 2) kann über diesen hinteren Eingang 7a in den Führungskanal 7 eingeschoben werden. Das vordere Ende und somit der vordere Ausgang 7b können in einen Kleinschnitt im zu operierenden Auge eingeführt werden und dazu die Intraokularlinse 14 aus der Injektorvorrichtung 1 in das Auge eingeschoben werden.

Wie aus der Darstellung in Fig. 1 und Fig. 2 zu erkennen ist, verjüngt sich der Führungskanal 7 von seinem hinteren Eingang 7a aus betrachtet zu seinem vorderen Ausgang 7b hin betrachtet, wobei dies insbesondere kontinuierlich erfolgt.

Wie gezeigt ist, umfasst die Injektorvorrichtung 1 zwei Verschließspitzen 15 und 16. Diese Verschließspitzen 15 und 16, welche auch als Verschließzinken oder Verschließkufen oder Verschließfinger bezeichnet werden können, erstrecken sich parallel zueinander und in Bezug zur Längsachse A beabstandet zueinander. Im Hinblick auf die Längsachse A sind die beiden Verschließspitzen 15 und 16 symmetrisch gegenüberliegend dazu positioniert und symmetrisch zueinander ausgebildet. Wie aus der Darstellung in Fig. 1 und Fig. 2 zu entnehmen ist, erstrecken sich die Verschließspitzen 15 und 16 im Aufnahmeraum 8. Sie münden an eine vordere Begrenzungswand 17, die den Aufnahmeraum 8 frontseitig begrenzt. Die Begrenzungswand 17 ist in der gezeigten Ausführung einstückig mit der Injektorspitze 2 gestaltet. Die Verschließspitzen 15 und 16 sind zum Verschließen von Deckelklappen der Kassette 6 gestaltet, wenn eine axiale Relativverschiebung zwischen den Verschließspitzen 15 und 16 und der Kassette 6 erfolgt.

Wie dazu in Fig. 1 und Fig. 2 in dem hier verdeutlichten Betriebszustand dargestellt ist, ist die Vorrichtung 9 mit dem Trägerschlitten 10 und der Kassette 6 am hinteren Ende des Aufnahmeraums 8 positioniert und somit ein positioneller Anfangszustand eingenommen. In dieser Position ist insbesondere noch keine Berührung zwischen den Verschließspitzen 15 und 16 und der Kassette 6 gegeben.

Die Verschließspitzen 15 und 16 sind analog ausgebildet, so dass die nachfolgende Erläuterung zur Verschließspitze 15, wie sie in Fig. 2 vergrößert dargestellt ist, auch für die Verschließspitze 16 gilt.

Die Verschließspitzen 15 und 16 erstrecken sich in axialer Richtung betrachtet weiter nach hinten als der hintere Eingang 7a der Injektorspitze 2.

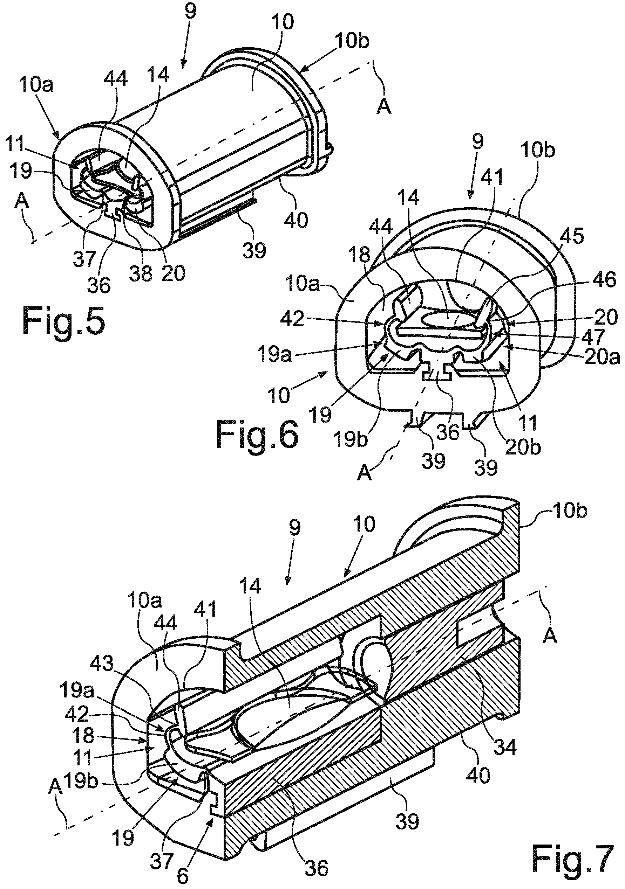

Wie insbesondere in Fig. 2 zu erkennen ist, ist die Kassette 6 so im Inneren 11 positioniert, dass zwischen einer Innenwand 18 des Trägerschlittens 10 und den Deckelklappen 19 und 20 (Fig. 5) der Kassette 6 ein Eingriffsraum 21 gebildet ist. In diesen Eingriffsraum 21 greifen die Verschließspitzen 15 und 16 ein, wenn eine axiale Relativverschiebung zwischen den Verschließspitzen 15 und 16 und der Vorrichtung 9 erfolgt.

Die Verschließspitze 15 umfasst einen hinteren ersten Längenabschnitt 22, der sich zu einem hinteren Ende 23 der Verschließspitze 15 verjüngt. Ein Oberseiteabschnitt 24 einer Oberseite des hinteren ersten Abschnitts 22 ist mit einem ersten S-förmigen Verlauf gestaltet, wobei dieser erste S-förmige Verlauf in einer Schnittebene, die sich in der x-z-Ebene erstreckt, gebildet ist. Dieser rampenartige Anstieg mit dem ersten S-förmigen Verlauf geht dann in einem vorderen zweiten Längenabschnitt 25 der Verschließspitze 15 in einen eben gestalteten Oberseitenabschnitt 26 über. Durch die Oberseitenabschnitte 24 und 26 ist ein Teil einer Führungsfläche 27 gebildet. Die Führungsfläche 27 ergänzt sich im Weiteren durch einen zweiten S-förmigen Verlauf einer Oberseite 28 im ersten hinteren Abschnitt 22. Dieser zweite S-förmige Verlauf ist ebenfalls in einer Schnittebene zu verstehen, die sich in der x-z-Ebene erstreckt.

Wie aus der Darstellung in Fig. 2 zu erkennen ist, geht dann auch dieser zweite S-förmige Verlauf des Oberseiteabschnitts 28 in einen ebenen Oberseitenabschnitt 29 im vorderen zweiten Abschnitt 25 der Verschließspitze 15 über.

Wie dazu in Fig. 2 zu erkennen ist, ist in Höhenrichtung und somit in z-Richtung der zweite S-förmige Verlauf und somit der Oberseitenabschnitt 28 tiefer liegend als der Oberseitenabschnitt 24. Ebenso ist der Oberseitenabschnitt 29 tiefer liegend als der Oberseitenabschnitt 26. Die Führungsfläche 27 wird somit insbesondere durch die Oberseitenabschnitt 24, 26, 28 und 29 gebildet.

Zwischen den Oberseitenabschnitten 24 und 28 ist zumindest teilweise über die Länge und somit über die Erstreckung in x-Richtung betrachtet ein gestufter Übergang 30 ausgebildet. Entsprechend ist auch ein gestufter Übergang 31 zwischen den Oberseitenabschnitten 26 und 29 gebildet. Wie gemäß Fig. 2 gezeigt ist, sind die tiefer liegenden Oberseitenabschnitte 28 und 29 näher zur Längsachse A angeordnet als die höher liegenden Oberseitenabschnitte 24 und 26. Die tiefer liegenden Oberseitenabschnitte 28 und 29 sind somit der Verschließspitze 16 zugewandt ausgebildet.

Das Injektorrohr 3 weist darüber hinaus auch noch einen Führungsschlitz 32 auf, der in einem Boden 33 des Injektorrohrs 3 ausgebildet ist. In diesem Führungsschlitz 32 ist die Vorrichtung 9 eingreifend angeordnet, so dass die axiale Relativverschiebung des Injektorrohrs 3 relativ zur Vorrichtung 9 und zum Kolbenrohr 4 entsprechend geführt ist und seitlich stabilisiert ist.

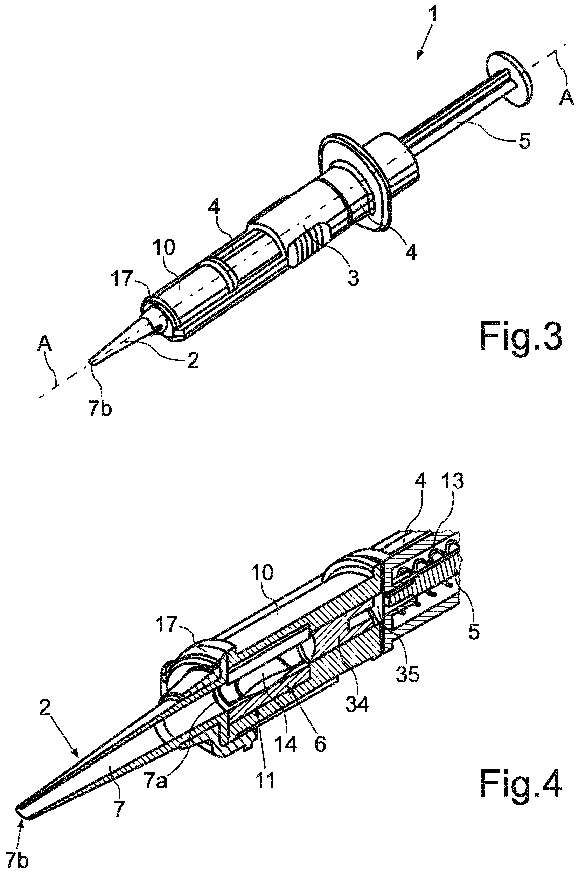

In Fig. 3 ist die Injektorvorrichtung 1 in einem zu Fig. 1 und Fig. 2 unterschiedlichen Betriebszustand gezeigt. Im Unterschied zu Fig. 1 und Fig. 2 ist hier der vollständig zurückgeschobene Zustand des Injektorrohrs 3 relativ zum Kolbenrohr 4 dargestellt. Die Vorrichtung 9 mit dem Trägerschlitten 10 ist bei dieser Ausgestaltung somit an der Begrenzungswand 17 insbesondere anschlagend angeordnet. Bei dieser Ausführung sind die Verschließspitzen 15 und 16 maximal in das Innere 11 eingeschoben positioniert.

Aufgrund der Formgebung der Verschließspitzen 15 und 16 wird auf diesem Bewegungsweg zwischen den Betriebszuständen gemäß Fig. 1 und Fig. 2 einerseits und Fig. 3 und Fig. 4 andererseits durch diese axiale Relativverschiebung automatisch ein Bewegen der Deckelklappen 19 und 20 der Kassette 6 um die Längsachse A bewirkt, so dass diese ausgehend von dem in Fig. 2 gezeigten vollständig geöffneten Zustand in den in Fig. 4 gezeigten vollständig geschlossenen Zustand übergeführt werden und dies automatisch erfolgt.

Dadurch ist die zunächst vorzugsweise in unverformtem Zustand in der Kassette 6 gelagerte Intraokularlinse 14 bei dem Betriebszustand gemäß Fig. 3 und Fig. 4 in der gewünschten vorgefalteten Endstellung in der Kassette 6 positioniert. Wie in Fig. 2 und Fig. 4 zu erkennen ist, umfasst der Trägerschlitten 10 im hinteren Bereich einen Kanal, in dem ein Ausschiebeelement 34, welches vorzugsweise aus einem elastischen verformbaren Material gebildet ist, angeordnet. Dieses auch als Dämpfungselement bezeichnete Bauteil wird beim Ausschieben der Intraokularlinse 14 aus der Kassette 6 durch den Kolben 5 in das Innere 11 geschoben, so dass der Kolben 5 nicht direkt mit der Intraokularlinse 14 in Berührung kommt. Dies hat dahingehend Vorteile, dass der sehr harte und steife Kolben 5 dann keine mechanische Beschädigung an der Intraokularlinse 14 hervorruft.

Wie in Fig. 2 und Fig. 4 zu erkennen ist, ist auch am hinteren Ende 10b ein Abdeckelement 35 angeordnet, welches auch eine Folie sein kann. Dieses Abdeckelement 35 wird dann durch den Kolben 5 durchstoßen und das Element 34 in das Innere 11 eingeschoben. Dies erfolgt gemäß der Darstellung in Fig. 4 jedoch erst dann, wenn einerseits der Betriebszustand gemäß Fig. 3 und Fig. 4 erreicht ist und dann der Kolben 5 in axialer Richtung nach vorne geschoben wird.

In Fig. 2 und Fig. 4 ist darüber hinaus auch ein Grundteil 36 der Kassette 6 zu erkennen, welches fest mit dem Trägerschlitten 10 verbunden ist. Dazu ist beispielsweise eine im Querschnitt T-förmige Gestalt des Grundteils 36 ausgebildet, so dass eine gewisse Verankerung der Kassette 6 in dem Trägerschlitten 10 erreicht ist. An dieses Grundteil 36 sind die beiden Deckelklappen 19 und 20 relativ dazu verschwenkbar angeordnet. Insbesondere ist die Kassette 6 einstückig aus Kunststoff ausgebildet und die Deckelklappen 19 und 20 sind über Filmscharniere 37 und 38 (Fig. 5) daran angeordnet.

In Fig. 4 ist der endgültige vorgefaltete Zustand der Intraokularlinse 14 in der Kassette 6 gezeigt, bevor diese Intraokularlinse 14 dann nachfolgend in den Führungskanal 7 eingeschoben wird.

In Fig. 5 ist eine perspektivische Darstellung eines Ausführungsbeispiels der Vorrichtung 9 gezeigt. Die röhrenförmige Ausgestaltung des Trägerschlittens 10 ist dargestellt. Darüber hinaus sind auch Führungskufen 39, die an einer Unterseite 40 des Trägerschlittens 10 ausgebildet sind, gezeigt. Mittels dieser Führungskufen 39 ist das Eingreifen in die Führungsbahn 32 ermöglicht.

In Fig. 6 ist eine weitere perspektivische Darstellung der Vorrichtung 9, wie sie in Fig. 5 gezeigt ist, dargestellt. Die Deckelklappen 19 und 20 sind in vollständig geöffnetem Zustand dargestellt. Die Innenwand 18 des Trägerschlittens 9, die das Innere 11 begrenzt, umfasst eine kuppelartige bzw. gewölbte Deckenseite 41, so dass ein Tunnel gebildet ist.

Im gezeigten Ausführungsbeispiel sind die Deckelklappen 19 und 20 in sich selbst verformbar. Insbesondere ist das Verformen definiert und somit gerichtet und selbständig durch den Schließvorgang bewirkt. In einer unverformten Grundstellung weisen diese Deckelklappen 19 und 20, wie es in Fig. 5 bis 7 gezeigt ist, einen bogenförmigen Klappenabschnitt 42 auf. An einem vorderen freien Ende 43 dieses bogenförmigen Klappenabschnitts 42 ist eine hammerkopfförmige Verdickung 44 ausgebildet. Analog ist dies bei der Deckelklappe 20 realisiert.

Die Deckelklappen 19 und 20 weisen Außenseiten 19a und 20a auf. Diese sind nicht glatt sondern mit unterschiedlichen Krümmungen ausgebildet. Dies ergibt sich durch unterschiedliche radiale Dicken von Längenbereichen der Deckelklappen in Ihren Längen in Richtung um die Achse A betrachtet. Die Deckelklappen weisen dazu neben den endseitigen Verdickungen 44 und 45 dünnere Klappenabschnitte 42 und 47 auf, an die dann andererseits wieder dickere Bereiche als nach außen gebildete Erhebungen 19b und 20b anschließen. Die Klappenabschnitte 42 und 47 sind in sich senkrecht zur Achse A reversibel wiederholbar verbiegbar, können jedoch auch nur zur definierten einmaligen und somit plastischen Verformung ausgebildet sein.

Das Grundteil 36 ist insbesondere starr und um die Längachse A nicht verformbar. Die insich-selbst-Verformbarkeit der Deckelklappen 19 und 20 bedeutet, dass sie ihre bogenförmige Struktur um die Längsachse A zerstörungsfrei verformen können, so dass die Bogenform verstärkt oder abgeschwächt werden kann. Dies erfolgt in Richtung der Längsachse betrachtet über die gesamte Länge der Deckelklappen 19 und 20 jedoch jeweils in gleicher Weise, so dass ein unerwünschtes Verdrillen um die Achse A verhindert ist.

Durch diese Möglichkeit, dass sich die Deckelklappen 19 und 20 zumindest bereichsweise entlang ihrer Längen (bemessen im Querschnitt senkrecht zur Achse A in Richtung um die Achse A) in Längenabschnitten in sich selbst senkrecht zur Achse A verformen können, wird das Vorfalten der Intraokularlinse 14 in der Kassette 6 begünstigt.

Der diesbezügliche Ablauf wird später noch erläutert.

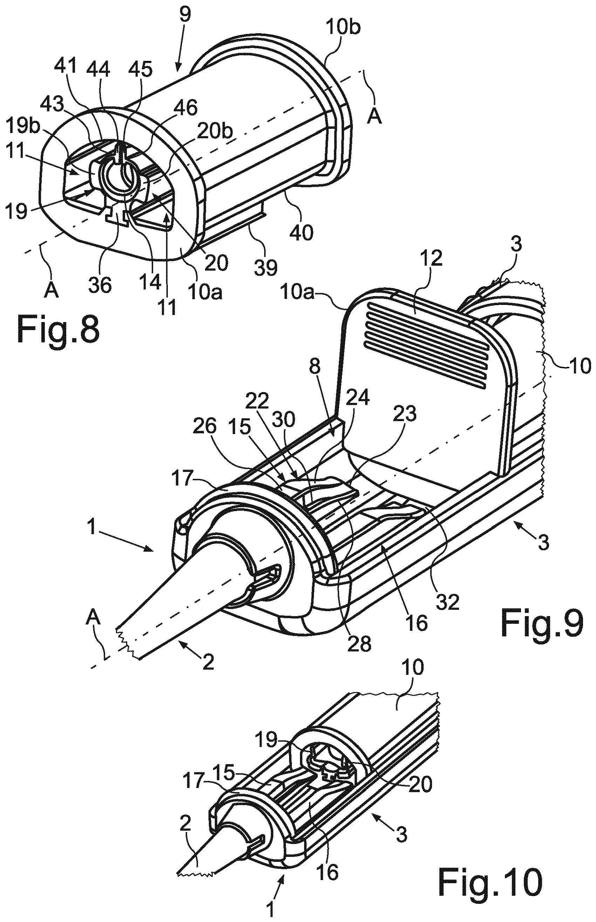

In Fig. 8 ist eine perspektivische Darstellung der Vorrichtung 9 gezeigt, bei der die Deckelklappen 19 und 20 im vollständig geschlossenen Zustand dargestellt sind. Die hammerkopfartige Verdickung 45, die am freien Ende 46 der Deckelklappe 20 angeordnet ist, ist ebenfalls zu erkennen.

In Fig. 9 ist eine vergrößerte Darstellung eines Ausschnitts von Fig. 1 gezeigt. Die Gestaltung der Verschließspitzen 15 und 16 ist zu erkennen. Es ist darüber hinaus auch zu erkennen, dass sich der Übergang 30 zwischen den Oberseitenabschnitten 24 und 28 vom Beginn des hinteren Abschnitts 22 bis zum hinteren Ende 23 abschwächt bzw. die Stufung abnimmt.

In Fig. 10 ist eine Darstellung des Ausschnitts gemäß Fig. 9 gezeigt, bei der ein weiterer Betriebszwischenzustand dargestellt ist und bei dem die Verschließspitzen 15 und 16 bereits bereichsweise in das Innere 11 eingeschoben sind. Die Kontaktierung der Führungsflächen 27 der Verschließspitzen 15 und 16 mit Außenseiten 19a, 20a der Deckelklappen 19 und 20 ist bereits erfolgt.

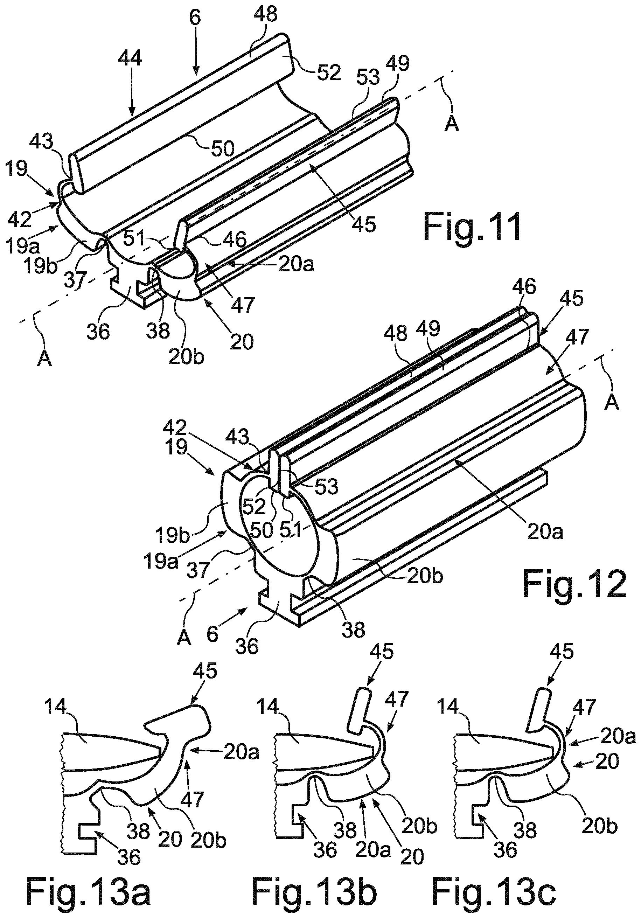

In Fig. 11 ist eine perspektivische Darstellung eines Ausführungsbeispiels der Kassette 6 gezeigt, wobei hier die Deckelklappen 19 und 20 im geöffneten Zustand gezeigt sind. Es ist zu erkennen, dass die bogenförmigen Klappenabschnitte 42 und 47 senkrecht zur Achse A verformbar sind, wobei deren radiale Dicke und somit senkrecht zur Achse A betrachtet kleiner ist, als die radialen Ausmaße der Verdickungen 44 und 45, sowie der Erhebungen 19b, 20b.

Darüber hinaus erstrecken sich die stegartigen Verdickungen 44 und 45 in radialer Richtung und somit senkrecht zur Achse A beidseits der Enden 43 und 46.

Die beiden hammerkopfartigen Verdickungen 44 und 45 weisen in radialer Richtung betrachtet äußere Enden 48 und 49 sowie innere Enden 50 und 51 auf. Zwischen diesen Enden 48 und 50 einerseits und 49 und 51 andererseits sind Anlageflächen 52 und 53 gebildet. Wie dazu in Fig. 12, in welcher eine perspektivische Darstellung der Kassette 6 mit vollständig geschlossenen Deckelklappen 19 und 20 gezeigt ist, zu erkennen ist, liegen diese Anlageflächen 52 und 53 vollflächig aneinander an.

In den Fig. 11 und 12 ist die röhrenförmige Gestalt der Kassette 6 zu erkennen.

In Fig. 13a bis 13c sind Frontansichten der unterschiedlichen Ausgestaltungen einer Kassette 6 mit eingebrachter Intraokularlinse 14 gezeigt, wobei hier insbesondere Unterschiede in den Querschnittausgestaltungen der Deckelklappen 20 dargestellt sind. Insbesondere sind unterschiedliche Querschnittformen der hammerkopfartigen Verdickungen 45 dargestellt. Dabei ergeben sich unterschiedliche Formgebungen derjenigen Teilbereiche der Hammerkopfform, die der Intraokularlinse 14 zugewandt sind.

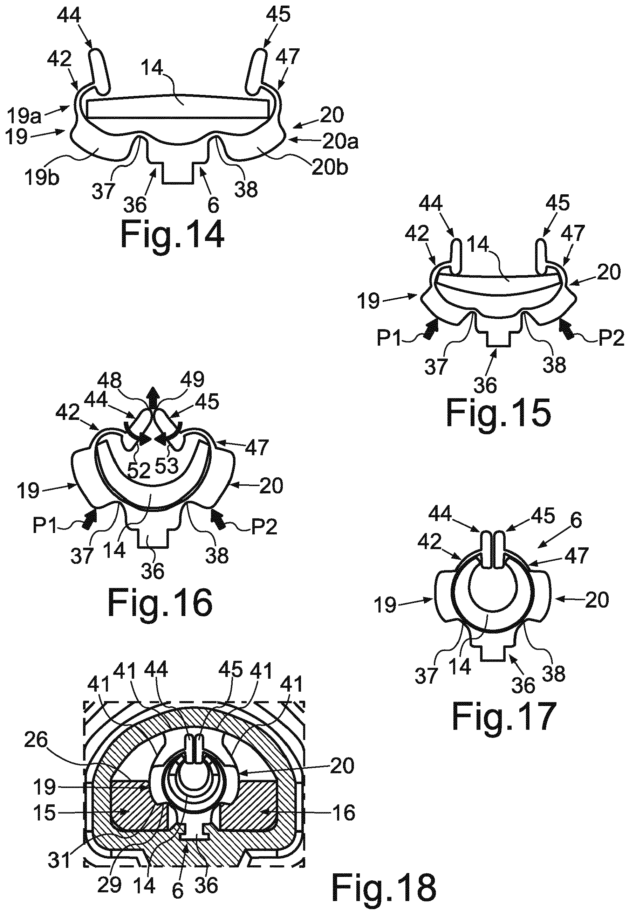

In den nachfolgenden Fig. 14 bis Fig. 17 wird das Szenario zum Vorfalten der Intraokularlinse 14 in der Kassette 6 näher erläutert.

Dazu ist in Fig. 14 der Grundzustand gezeigt, bei dem die Deckelklappen 19 und 20 vollständig geöffnet sind und die Deckelklappen 19 und 20 in sich in einem zwar gebogenen aber unverformten Grundzustand gezeigt sind, wobei dabei die Grundform der bogenförmigen Klappenabschnitte 42 und 47 gezeigt ist. Die Intraokularlinse 14 ist in diesem Zustand noch nicht gebogen und liegt an den Innenseiten der Deckelklappen 19 und 20 auf.

Ausgehend von diesem Zustand bei einem dann erfolgenden axialen relativen Verschieben zueinander zwischen den Verschließspitzen 15 und 16 und der Kassette 6 ein Berühren der Führungsflächen 27 der Verschließspitzen 15 und 16 an den Außenseiten 19a, 20a der Deckelklappen 19 und 20 und heben diese gemäß den Pfeilen P1 und P2 in Fig. 15 an, so dass diese um die senkrecht zur Figurenebene stehende Längsachse A verschwenkt werden.

Da die Klappenabschnitte 42 und 27 einen kleineren Radius aufweisen als der jeweilige Rest der Deckeklappen 19 und 20, wird bei einem weiteren Verschwenken um die Längsachse A werden die Verdickungen 44 und 45 so nach innen geführt, dass sie auf der Intraokularlinse 14 aufsitzen und somit zumindest durch die Verdickungen 44 und 45 Faltstabilisatoren gebildet werden. Die Intraokularlinse 14 ist zumindest ab dann in gewissem Maße eingespannt und bei einer weiteren axialen Relativverschiebung zwischen der Kassette 6 und den Verschließspitzen 15 und 16 erfolgt das weitere Aufeinanderzubewegen der Deckelklappen 19 und 20, so dass dann die weitere Zwischenposition gemäß Fig. 16 erreicht wird. In dieser kontaktieren sich zunächst nur die äußeren Enden 48 und 49. Eine mechanische Berührung ist zunächst nur an diesen Enden 48, 49 erreicht und die Anlageflächen 52 und 53 sind im Übrigen noch berührungslos angeordnet.

Diese Verform- und Faltabläufe, wie sie in den Fig. 14 bis Fig. 15 bisher erläutert sind, sind wesentliche Voraussetzung für die gewünschte definierte Vorfaltung der Linse 14. Durch die spezifische Formgebung des Inneren 11 des Trägerschlittens 10 wird dann im Weiteren ermöglicht, dass sich ausgehend von dem Zwischenzustand in Fig. 16 der geschlossene Zustand der Deckelklappen 19 und 20 automatisch einstellen kann. Aufgrund der Insichverformung der Deckelklappen 19 und 20, stellt sich ein gewisser Spannungszustand in den Deckelklappen 19 und 20 ein. Aufgrund der erreichten Anlage der äußeren Enden 48 und 49 kann bei einer weiteren Bewegung der Deckelklappen 19 und 20 aufeinander zu im Selbstautomatismus ein Anlegen der Anlageflächen 52 und 53 automatisch erreicht werden und die gegenseitige Abstützung der Verdickungen 44 und 45 ein nach außen Bewegen, insbesondere ein nach außen Schnappen dieser Enden 44 und 45 erreicht werden. Dadurch wird dann der nach innen gebogene Grundzustand der Klappenabschnitte 42 und 47 automatisch verformt und nach außen gebogen, insbesondere aufgrund der Abstützkräfte zwischen den Verdickungen 44 und 45 ein nach außen Schnappen bewirkt, und der sich selbst stabilisierende und selbst haltende Endzustand gemäß Fig. 17 erreicht. Dort liegen die Anlageflächen 52 und 53 vollflächig aneinander an und es ist im Querschnitt betrachtet ein im Wesentlichen symmetrischer und runder Aufnahmeraum für die Intraokularlinse 14 gebildet, der lediglich durch die hammerkopfartigen Verdickungen 44 und 45 unterbrochen ist. Zumindest ab dem Zustand gemäß Fig. 16 ist die Deckenseite 41 mit einem nach oben entsprechend ausgebildeten Freiraum versehen, so dass dann ein nach oben Schnappen der Verdickungen 44 und 45 ermöglicht ist. Dies ist in der Schnittdarstellung gemäß Fig. 18 gezeigt, bei der der Endzustand der Kassette 6 mit der Intraokularlinse 14, wie in Fig. 17 gezeigt ist, im in die Injektorvorrichtung 1 und somit auch in die Vorrichtung 9 angeordneten Zustand gezeigt ist.

Wie in Fig. 18 zu erkennen ist, ist die Formgebung der Außenseite 19a, 20a der Deckelklappen 19 und 20 vorzugsweise derart, dass ein großflächiges, insbesondere vollflächiges und relativ passgenaues Anliegen an Führungsflächen 27 der Verschließspitzen 15 und 16 zumindest in dieser Endlage ausgebildet ist.

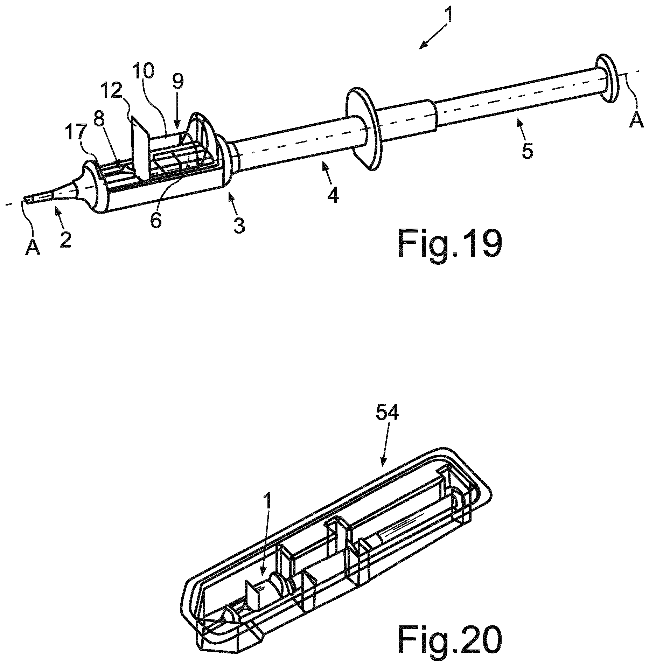

In Fig. 19 ist in einer perspektivischen Darstellung ein weiteres Ausführungsbeispiel einer Injektorvorrichtung 1 gezeigt. Im Unterschied zur Darstellung in dem bisherigen Ausführungsbeispiel ist hier vorgesehen, dass das Injektorrohr 3 integral mit dem Kolbenrohr 4 ausgebildet ist. Dies bedeutet, die beiden Rohre 3 und 4 sind ortsfest miteinander verbunden, insbesondere einstückig ausgebildet.

Auch hier ist der nach oben offene Aufnahmeraum 8 gebildet und die Vorrichtung 9 mit einer Kassette 6 und einem Trägerschlitten 10 eingesetzt.

In Fig. 20 ist ein Ausführungsbeispiel einer Verpackungs- und Transportvorrichtung 54 gezeigt. Der diesbezügliche Behälter ist zur Aufnahme der Injektorvorrichtung 1, wie er in den Fig. 1 bis 4 erläutert wurde, oder zur Aufnahme der Injektorvorrichtung 1 gemäß Fig. 19 ausgebildet. Er ist mit einer entsprechenden sterilen Flüssigkeit gefüllt, wobei auch hier bereits eine entsprechende sterile Flüssigkeit in der Kassette 6 zur Umspülung der Linse 14 vorgesehen ist. Der Behälter ist dann durch eine Abdeckung, wie beispielsweise eine Folie, steril verschlossen und kann in dieser kompakten Ausgestaltung dann von dem Hersteller zum medizinischen Personal geliefert werden. Das medizinische Personal muss dann nur noch den vollständig beladenen Injektor bzw. Injektorvorrichtung 1 aus der Vorrichtung 54 entnehmen und keinerlei weitere Beladung der Injektorvorrichtung 1 mit einer Kassette oder gegebenenfalls die Entnahme einer Intraokularlinse 14 aus einem weiteren separat angelieferten Behälter durchführen. Dadurch wird die Handhabung wesentlich vereinfacht und Fehler beim Beladen können vermieden werden.

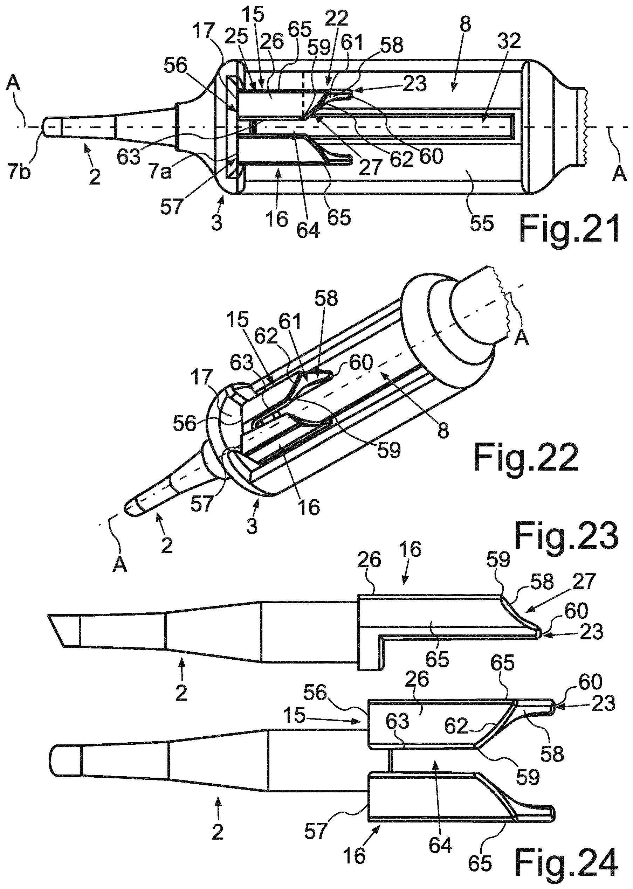

In Fig. 21 ist eine Draufsicht auf das Injektorrohr 3 mit der Injektorspitze 2 gezeigt, wobei nur der vordere Bereich mit dem Aufnahmeraum 8 und entnommener Vorrichtung 9 dargestellt ist. In einem Boden 55 des Injektorrohrs 3 ist die Führungsbahn bzw. der Führungsschlitz 32 zu erkennen. Die Verschließspitzen 15 und 16 sind dargestellt und die beabstandete Anordnung und Anbindung an die Begrenzungswand 17 mit vorderen Enden 56 und 57 ist gezeigt.

Bei dieser Ausgestaltung ist im Unterschied zu dem bisher erläuterten Ausführungsbeispiel vorgesehen, dass ein hinterer Abschnitt 22, der sich bis zur gestrichelten Linie erstreckt, sich verjüngend ausbildet. Dieser hintere Abschnitt 22 umfasst einen hinteren Führungsflächenabschnitt 58 der Führungsfläche 27, welcher steilkurvenartig ausgebildet ist. Dies bedeutet, dass er nicht in einer Ebene sich erstreckt, sondern gekrümmt und gewunden im Raum gebildet ist. Dieser steilkurvenartige Führungsflächenabschnitt 58 erstreckt sich von einem hinteren Ende 23 der Verschließspitze 15 bis zu einem vorderen Ende 59. Er erstreckt sich somit über die gesamte Länge des hinteren Abschnitts 22 der Verschließspitze 15. Dieser Führungsflächenabschnitt 58 ist darüber hinaus so gestaltet, dass ein hinteres Flächenende 60 dünner bzw. schmäler ist, als ein Mittenstück 61, so dass ausgehend von diesem hinteren Ende 60 dieser Flächenabschnitt 58 sich kontinuierlich aufweitet und vergrößert, um dann ausgehend von der maximalen Breite sich bis zum vorderen Ende 59 wieder verjüngt. Es wird somit quasi eine Art Bumerangform gebildet, die darüber hinaus auch noch eine gewisse Windung nach Art eines helixförmigen Abschnitts bildet. Auch hier gilt die Erläuterung zur Verschließspitze 15 analog für die Verschließspitze 16, da diese gleiche ausgebildet ist und eine symmetrische Gestaltung zur Längsachse A erzeugt ist.

Anschließend an den hinteren Längenabschnitt 22 der Verschließspitze 15 ist der vordere Längenabschnitt 25 gebildet. Dieser umfasst eine ebene Oberseite 26, die an einem oberen Rand 62 des steilkurvenartigen Führungsflächenabschnitts 58 an dem vorderen ersten Abschnitt 22 angrenzt.

Die Führungsfläche 27 weist neben dem steilkurvenartigen Führungsflächenabschnitt 58 einen vorderen Führungsflächenabschnitt 63 auf, der parallel zur Längsachse A verläuft. Wie aus der Darstellung in Fig. 21 zu erkennen ist, ist ein radialer Abstand und somit ein Abstand senkrecht zur Längsachse A zwischen den hinteren Enden 60 der Spitzen 15 und 16 größer als ein entsprechender Abstand zwischen den vorderen Enden 59 des vorderen Führungsflächenabschnitts 58, wobei dieser dann erreichte kleinere Abstand an den vorderen Enden 59 gleich dem Abstand zwischen den vorderen Führungsflächenabschnitten 63 entspricht, der dann insbesondere über die gesamte Länge der vorderen Führungsflächenabschnitte 63 konstant bleibt.

Durch diese Führungsflächenabschnitte 63 und deren Beabstandung ist ein Führungsschlitz 64 gebildet. In diesem werden die im Wesentlichen geschlossenen Deckelklappen 19 und 20 der Kassette 6 geführt und in ihrer Position gehalten, bis die Kassette 6 die für die Axialverschieberichtung erreichte Endlage eingenommen hat.

In Fig. 22 ist eine perspektivische Darstellung der Fig. 21 gezeigt.

In Fig. 23 ist eine Seitenansicht gezeigt, bei der lediglich die Injektorspitze 2 und die Verschließspitze 16 gezeigt sind. Diesbezüglich ist die Kontur des äußeren Rands der Führungsfläche 27 im Bereich des Führungsflächenabschnitts 58 dargestellt. Eine Außenseite 65, die an die Oberseite und den Führungsflächenabschnitt 58 angrenzend ist, ist ebenfalls dargestellt.

In Fig. 24 ist eine Draufsicht auf die Komponenten in Fig. 23 gezeigt, wobei bezüglich der Merkmale und Ausgestaltungen auf die Erläuterungen zu Fig. 21 bis 23 verwiesen wird.

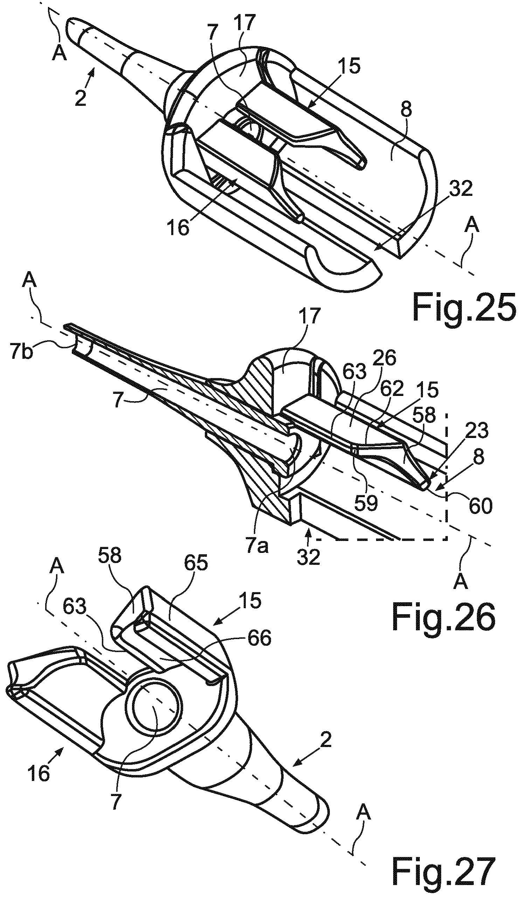

In Fig. 25 ist eine weitere perspektivische Darstellung der Ausführungen in Fig. 21 bis 24 gezeigt.

Die Lage der Verschließspitzen 15 und 16 relativ zum Führungskanal 7 ist gezeigt.

Eine genauere Ansicht ist dazu in der Schnittdarstellung in Fig. 26 zu erkennen, wobei hier die Schnittebene die Längsachse A umfasst.

In den Fig. 25 bis 27 ist darüber hinaus die Form und Krümmung des Führungsflächenabschnitts 58 im dreidimensionalen Raum noch detaillierter zu erkennen. Die sichel- bzw. bumerangartige Flächenform mit der zusätzlichen unebenen Windung zur Gestaltung einer steilkurvenartigen Formgebung ist gezeigt.

Darüber hinaus ist auch die gewölbte bzw. kuppelartige Form einer Unterseite 66, die an die Außenseite 65 und die Oberseite 26 sowie den Abschnitt 58 angrenzt, gezeigt. Die Unterseite 66 geht in den vorderen Führungsflächenabschnitt 63 über, der dann in die Oberseite 26 mündet. Durch diese genannten Flächenteile wird eine Verschließspitze 15 bzw. 16 gebildet.

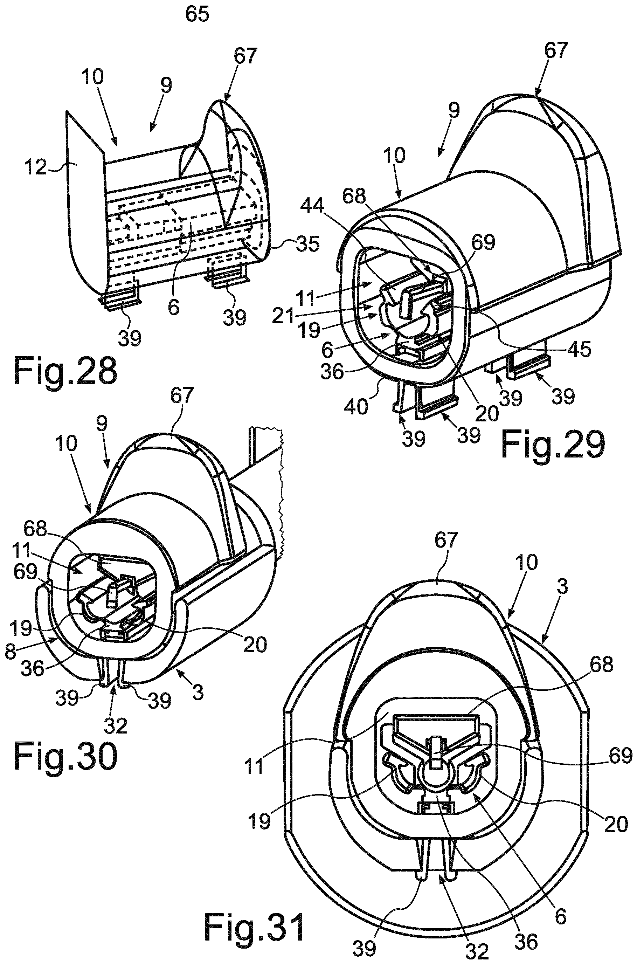

In Fig. 28 ist eine perspektivische Darstellung des Ausführungsbeispiels einer Vorrichtung 9, die eine Kassette 6 und einen Trägerschlitten 10 aufweist, gezeigt.

In Fig. 29 ist dazu eine perspektivische Ansicht dargestellt, wobei die frontseitige Abdeckung in Form des Abdeckelements 12 bereits abgenommen ist.

Bei dieser Ausführung umfasst der Trägerschlitten 10 ein nach oben stehendes plattenartiges Griffteil 67, an welchem ein Nutzer angreifen kann, um die Vorrichtung 9 in Richtung der Längsachse A relativ zu den Verschließspitzen 15 und 16 im Aufnahmeraum 8 verschieben zu können.

In der Darstellung gemäß Fig. 29 sind die mehreren Führungselemente in Form von Kufen bzw. Rastelementen 39 dargestellt, die an der Unterseite 40 befestigt sind.

Bei diesem Ausführungsbeispiel ist die Kassette 6 neben dem Grundteil 36 und den beiden flügelartigen Deckelklappen 19 und 20 zusätzlich mit einem Sicherungsdeckel 68 ausgebildet. Der Sicherungsdeckel 68 ist vorzugsweise ebenfalls einstückig mit den anderen Komponenten der Kassette 6 ausgebildet. Ein derartiger Sicherungsdeckel 68 kann auch bei den anderen bisher erläuterten Ausführungen von Kassetten 6 vorgesehen sein.

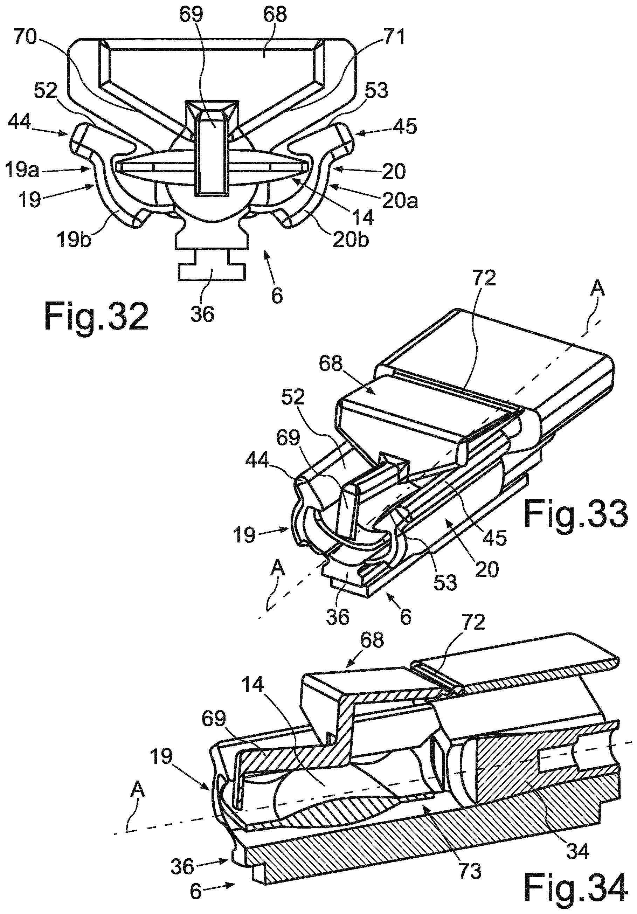

Der Sicherungsdeckel 68 ist relativ schwenkbar zum Grundteil 36 daran befestigt, wobei dazu insbesondere eine Verbindung über ein Filmscharnier 72 (Fig. 33) vorgesehen ist.

Im geöffneten Zustand der Deckelklappen 19 und 20 ist der Sicherungsdeckel 68 geschlossen. Er weist an seinem vorderen Ende einen hakenförmigen Sicherungsbügel 69 auf, so dass die Intraokularlinse 14 weder nach oben noch nach vorne aus der Kassette 6 herausfallen oder herausrutschen kann.

In Fig. 30 ist eine weitere perspektivische Darstellung der Vorrichtung 9 im in den Aufnahmeraum 8 eingesetzten Zustand gezeigt. Die verrastete Anordnung ist zu erkennen, wobei dazu die Elemente 39 in dem Führungsschlitz 32 angeordnet sind und sich hindurch erstrecken, so dass mit den Elementen 39 ein Hintergreifen der Außenseite des Injektorrohrs 3 erfolgt. Die axiale Verschiebung ist dadurch einfachst möglich und dennoch ein Verrutschen oder Verkanten in eine andere Raumrichtung verhindert. Durch die Flexibilität der Elemente 39 kann ein einfaches Einstecken und Verrasten der Vorrichtung 9 in den Aufnahmeraum 8 und den Führungsschlitz 32 erfolgen.

In Fig. 31 ist eine Frontansicht der Darstellung in Fig. 30 gezeigt. Es ist diesbezüglich die Lage der Kassette 6 im Inneren 11 zu erkennen.

In Fig. 32 ist eine Ansicht von vorne auf die Kassette 6, wie sie in Fig. 28 bis 31 erläutert wurde, gezeigt. Die Intraokularlinse 14 ist in der Grundposition darin angeordnet.

Ausgehend von der Darstellung in Fig. 32 kann der Sicherungsdeckel 68 automatisch angehoben und geöffnet werden. Dazu wird gemäß der Darstellung in Fig. 33 ein Verschwenken der Deckelklappen 19 und 20 um die Längsachse A bewirkt, wobei dies durch die bereits geschilderten Szenarien mit den Verschließspitzen 15 und 16 erfolgt. Dabei wird dann während dieser Verschwenkung der Deckelklappen 19 und 20 ein Kontaktieren der Verdickungen 44 und 45 mit dem Sicherungsdeckel 68 erreicht. Dieser umfasst dazu schräge Anhebeelemente, insbesondere Anhebeflanken 70 und 71. Bei weiterem Schließen der Deckelklappen 19 und 20 gleiten die Verdickungen 44 und 45 an diesen schrägen Anhebeflanken 70 und 71 entlang, wodurch der Sicherungsdeckel 68 angehoben wird, wie dies in Fig. 33 verdeutlicht ist. Das Filmscharnier 72 ist dabei horizontal orientiert, so dass der Deckel 68 um diese Achse schwenkbar nach oben angehoben wird, wie dies durch die Pfeile dargestellt ist.

In Fig. 34 ist eine Schnittdarstellung der perspektivischen Ansicht in Fig. 33 gezeigt. Die Intraokularlinse 14 ist dabei in einem Innenraum 73, der durch die Deckelklappen 19 und 20 und das Grundteil 36 im geschlossenen Zustand der Deckelklappen 19 und 20 begrenzt wird, angeordnet.

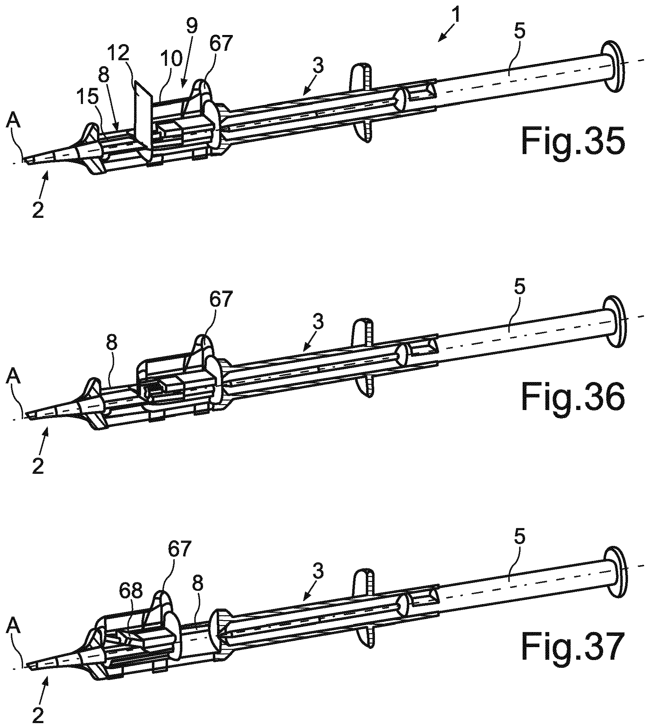

In Fig. 35 ist eine perspektivische Darstellung der Injektorvorrichtung 1 in einem Betriebszustand gezeigt, in dem die Vorrichtung 9 in einer hinteren Grundstellung in dem Aufnahmeraum 8 angeordnet ist.

Ausgehend von diesem Zustand wird dann im Nachfolgenden die frontseitige Abdeckung in Form des Abdeckelements 12 durch einen Nutzer entfernt, wie dies in Fig. 36 gezeigt ist. Im Nachfolgenden wird dann die Vorrichtung 9 mit dem Trägerschlitten 10 und der Kassette 6 zur Injektorspitze 2 hin axial nach vorne geschoben, bis sie die vordere Endposition gemäß der Darstellung in Fig. 37 erreicht hat. Während dieses Verschiebevorgangs zwischen den Positionen in Fig. 36 und Fig. 37 wird das automatische Schließen der Kassette 6 erreicht, da die Verschließspitzen 15 und 16 in das Innere 11 des Trägerschlittens 10 eingreifen und die Deckelklappen 19 und 20 anheben und schließen. Dabei wird die Intraokularlinse 14 in der Kassette 6 in einen vorgefalteten Zustand verbracht. Wie in Fig. 37 zu erkennen ist, ist dann auch automatisch der Sicherungsdeckel 68 angehoben.

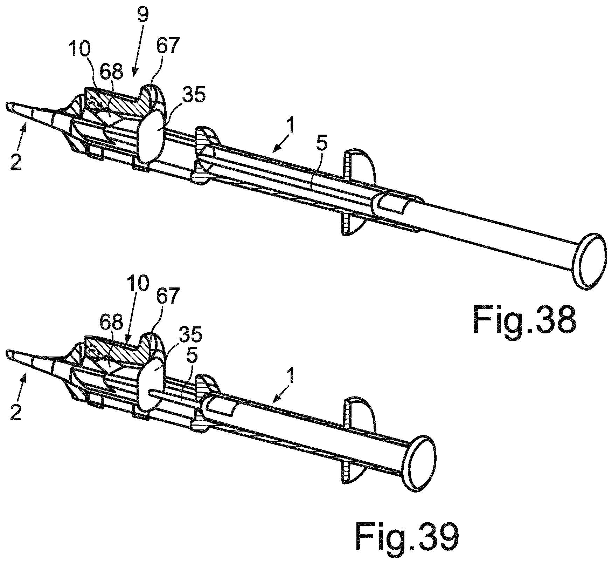

In einem weiteren Schritt wird dann gemäß der Darstellung in Fig. 38 der Kolben 5 nach vorne geschoben, bis er das hintere Abdeckelement 35 kontaktiert. Dieses ist insbesondere so ausgebildet, dass es durch den Kolben 5 durchstoßen werden kann, wie dies in Fig. 39 gezeigt ist. Es wird dann das elastische Element 34 durch den Kolben 5 nach vorne geschoben und die Intraokularlinse 14 kontaktiert und dann aus der Kassette 6 in den Führungskanal 7 eingeschoben. Dort wird sie dann weiter gefaltet, da sich der Führungskanal 7 bis zum Ausgang 7b verjüngt. In einer dann maximal gefalteten Stellung wird dann die Intraokularlinse 14 aus der Injektorspitze 2 ausgeschoben und ins Auge eingeschoben.

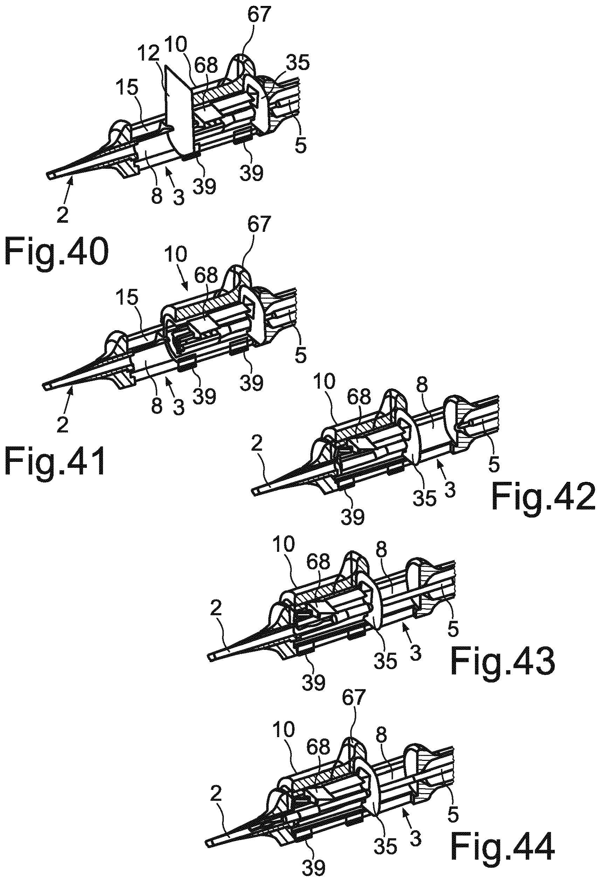

In den Fig. 40 bis 44 sind die anhand der Fig. 35 bis 39 erläuterten Betriebszustände nochmals dargestellt, wobei jeweils nur der Bereich der Injektorvorrichtung 1 gezeigt ist, der ab dem Aufnahmeraum 8 nach vorne ausgebildet ist.

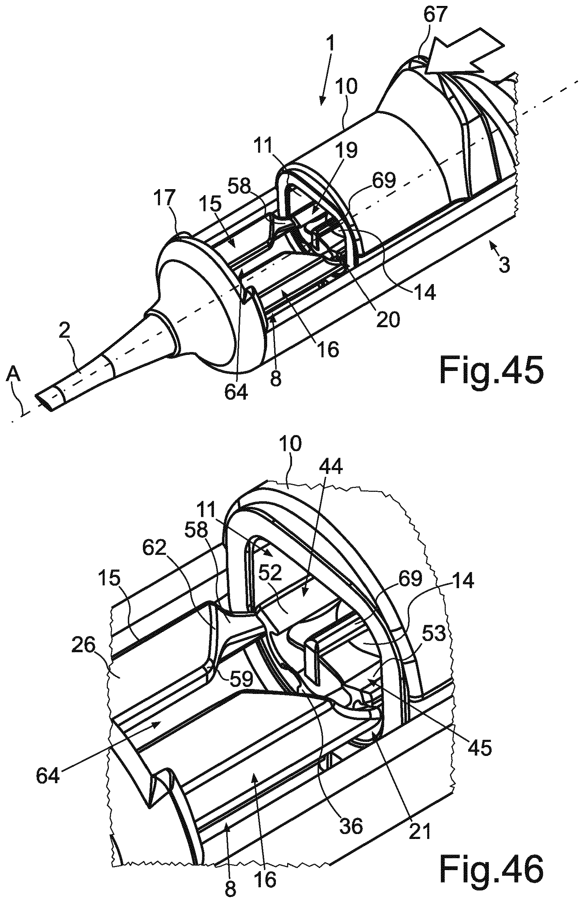

In Fig. 45 ist eine weitere perspektivische Darstellung von Teilkomponenten der Injektorvorrichtung 1 gezeigt, wobei hier ein Zustand gezeigt ist, bei dem die Verschließspitzen 15 und 16 gerade in das Innere 11 sich erstrecken und die Deckelklappen 19 und 20 an den Außenseiten 19a, 20a gerade berühren, um sich im Weiteren dann um die Achse A zu verschwenken und in die Schließposition zu verbringen.

In Fig. 46 ist dazu ein weiterer vergrößerter Ausschnitt der Darstellung in Fig. 45 gezeigt. Insbesondere wird hier auch das Angreifen der Verschließspitzen 15 und 16 an den Außenseiten 19a, 20a der Deckelklappen 19 und 20, insbesondere und vorzugsweise an den Außenseiten der Verdickungen 44 und 45 gezeigt. Durch die spezifische Formgebung der Verschließspitzen 15 und 16, insbesondere der Führungsflächen 27, die an die Formgebung der Außenseite der Deckelklappen 19 und 20 angepasst ist, kann ein optimaler Schwenkvorgang der Deckelklappen 19 und 20 ohne ein seitliches Vorbeirutschen erreicht werden.

## Patentansprüche

1. Injektorvorrichtung (1) zum Einführen einer Intraokularlinse (14) in ein Auge, welche eine Injektorspitze (2) mit einem durchgehenden Führungskanal (7) für die Intraokularlinse (14) aufweist, welcher einen hinteren Eingang (7a) und einen vorderen Ausgang (7b) aufweist, wobei
die Injektorvorrichtung (1) zumindest zwei Verschließspitzen (15, 16) aufweist, welche zum Verschließen einer die Intraokularlinse (14) aufnehmenden Kassette (6) ausgebildet sind, und welche sich in Richtung der Längachse (A) der Injektorvorrichtung (1) axial weiter nach hinten erstrecken als der hintere Eingang (7a), wobei die Injektorvorrichtung (1) eine Kassette (6) zur Aufnahme der Intraokularlinse (14) aufweist, und die Kassette (6) ein Grundteil (36) umfasst, an dem relativ zum Grundteil (36) um die Längsachse (A) der Injektorvorrichtung (1) schwenkbar Deckelklappen (19, 20) angeordnet sind,
**dadurch gekennzeichnet, dass**
die Verschließspitzen (15, 16) und die Kassette (6) so angeordnet sind, dass bei einer axialen Relativverschiebung zwischen der Kassette (6) und den Verschließspitzen (15, 16) die Verschließspitzen (15, 16) mit freien hinteren Längenabschnitten (22) Außenseiten von geöffneten Deckelklappen (19, 20) kontaktieren und bei einem weiteren axialen Verschieben zwischen der Kassette (6) und den Verschließspitzen (15, 16) relativ zueinander die Deckelklappen (19, 20) automatisch durch die Verschließspitzen (15, 16) um die Längsachse (A) der Injektorvorrichtung (1) verschwenkbar und schließbar sind, wobei die freien hinteren Längenabschnitte (22) der Verschließspitzen (15, 16) verjüngt ausgebildet sind und jeweils eine gebogene Führungsfläche (27) aufweisen, wobei die Führungsfläche (27) einen steilkurvenartigen hinteren Abschnitt (58) aufweist, wobei der steilkurvenartige hintere Abschnitt (58) ein dünnes hinteres Ende (60) aufweist, welches sich bis zu einer Mittendicke (61) kontinuierlich verbreitert und dann von der Mittendicke (61) sich kontinuierlich wieder bis zu einem vorderen Ende (59) dieses hinteren Abschnitts (58) verdünnt.

2. Injektorvorrichtung (1) nach Anspruch 1, **dadurch gekennzeichnet, dass** sich die Verschließspitzen (15, 16) beabstandet und parallel zueinander in Richtung der Längsachse (A) erstrecken und/oder die Verschließspitzen (15, 16) symmetrisch zueinander zur Längsachse (A) ausgebildet und angeordnet sind.

3. Injektorvorrichtung (1) nach Anspruch 1 oder 2, **dadurch gekennzeichnet, dass** der steilkurvenartige hintere Abschnitt (58) in einen parallel zur Längsachse (A) sich erstreckenden vorderen Abschnitt (63) der Führungsfläche (27) mündet.

4. Injektorvorrichtung (1) nach einem der vorhergehenden Ansprüche, **dadurch gekennzeichnet, dass** die Führungsflächen (27) der Spitzen (15, 16) jeweils einen vorderen Abschnitt (26, 63) aufweisen, wobei zumindest zwischen den vorderen Abschnitten (26, 63) ein Führungsschlitz (64) zum Führen in axialer Richtung für Deckelklappen (19, 20) der Kassette (6) ausgebildet ist.

## Claims

1. Injector apparatus (1) for introducing an intraocular lens (14) into an eye, which has an injector tip (2) with a continuous guide channel (7) for the intraocular lens (14), said guide channel (7) having a rear inlet (7a) and a front outlet (7b), wherein
the injector apparatus (1) has at least two closure tips (15, 16) which are configured to close a cassette (6) holding the intraocular lens (14), and which extend axially further to the rear in the direction of the longitudinal axis (A) of the injector apparatus (1) than the rear inlet (7a), wherein the injector apparatus (1) has a cassette (6) for holding the intraocular lens (14), and the cassette (6) comprises a base part (36) on which cover flaps (19, 20) that are pivotable about the longitudinal axis (A) of the injector apparatus (1) relative to the base part (36) are arranged,
**characterized in that**
the closure tips (15, 16) and the cassettes (6) are arranged such that, in the event of an axial relative displacement between the cassette (6) and the closure tips (15, 16), the closure tips (15, 16) come into contact, by way of free rear length portions (22), with outer sides of open cover flaps (19, 20), and in the event of further axial displacement between the cassette (6) and the closure tips (15, 16) relative to one another, the cover flaps (19, 20) are pivotable automatically about the longitudinal axis (A) of the injector apparatus (1) by the closure tips (15, 16) and closable,
wherein the free rear length portions (22) of the closure tips (15, 16) are formed in a tapered manner and each have a curved guide face (27), wherein the guide face (27) has a rear portion (58) in the form of a steep curve, wherein the rear portion (58) in the form of a steep curve has a thin rear end (60) which widens continuously to a central thickness (61) and then narrows continuously from the central thickness (61) again as far as a front end (59) of this rear portion (58).

2. Injector apparatus (1) according to Claim 1, **characterized in that** the closure tips (15, 16) extend in the direction of the longitudinal axis (A) in a manner spaced apart from and parallel to one another, and/or the closure tips (15, 16) are formed and arranged symmetrically to one another with respect to the longitudinal axis (A).

3. Injector apparatus (1) according to Claim 1 or 2, **characterized in that** the rear portion (58) in the form of a steep curve leads into a front portion (63), extending parallel to the longitudinal axis (A), of the guide face (27).

4. Injector apparatus (1) according to one of the preceding claims, **characterized in that** the guide faces (27) of the tips (15, 16) each have a front portion (26, 63), wherein, at least between the front portions (26, 63), a guide slot (64) for guidance in an axial direction for cover flaps (19, 20) of the cassette (6) is formed.

## Revendications

1. Dispositif injecteur (1) pour l'introduction d'une lentille intraoculaire (14) dans un oeil, qui présente une pointe d'injecteur (2) avec un canal de guidage continu (7) pour la lentille intraoculaire (14), lequel présente une entrée arrière (7a) et une sortie avant (7b),
le dispositif injecteur (1) présentant au moins deux pointes de fermeture (15, 16) qui sont réalisées pour fermer une cassette (6) recevant la lentille intraoculaire (14) et qui s'étendent dans la direction de l'axe longitudinal (A) du dispositif injecteur (1) axialement plus vers l'arrière que l'entrée arrière (7a), le dispositif injecteur (1) présentant une cassette (6) pour recevoir la lentille intraoculaire (14), et la cassette (6) comprenant une partie de base (36) sur laquelle sont disposés des volets de recouvrement (19, 20) pouvant pivoter par rapport à la partie de base (36) autour de l'axe longitudinal (A) du dispositif injecteur (1),
**caractérisé en ce que**
les pointes de fermeture (15, 16) et la cassette (6) sont disposées de telle sorte que dans le cas d'un déplacement axial relatif entre la cassette (6) et les pointes de fermeture (15, 16), les pointes de fermeture (15, 16) viennent en contact avec leurs portions longitudinales arrière libres (22) avec des côtés extérieurs de volets de recouvrement ouverts (19, 20) et dans le cas d'un déplacement axial supplémentaire entre la cassette (6) et les pointes de fermeture (15, 16) l'une par rapport à l'autre, les volets de recouvrement (19, 20) puissent être fermés et pivotés automatiquement autour de l'axe longitudinal (A) du dispositif injecteur (1) par les pointes de fermeture (15, 16), les portions longitudinales arrière libres (22) des pointes de fermeture (15, 16) étant réalisées de manière à se rétrécir et présentant à chaque fois une surface de guidage courbe (27), la surface de guidage (27) présentant une portion arrière (58) de type à courbure raide, la portion arrière (58) de type à courbure raide présentant une extrémité arrière mince (60) qui s'élargit en continu jusqu'à une épaisseur centrale (61) puis qui se rétrécit depuis l'épaisseur centrale (61) en continu à nouveau jusqu'à une extrémité avant (59) de cette portion arrière (58).

2. Dispositif injecteur (1) selon la revendication 1, **caractérisé en ce que** les pointes de fermeture (15, 16) s'étendent à distance et parallèlement l'une à l'autre dans la direction de l'axe longitudinal (A) et/ou les pointes de fermeture (15, 16) sont réalisées et disposées de manière symétrique l'une par rapport à l'autre par rapport à l'axe longitudinal (A).

3. Dispositif injecteur (1) selon la revendication 1 ou 2, **caractérisé en ce que** la portion arrière (58) de type à courbure raide débouche dans une portion avant (63) de la surface de guidage (27) s'étendant parallèlement à l'axe longitudinal (A).

4. Dispositif injecteur (1) selon l'une quelconque des revendications précédentes, **caractérisé en ce que** les surfaces de guidage (27) des pointes (15, 16) présentent à chaque fois une portion avant (26, 63), une fente de guidage (64) étant réalisée au moins entre les portions avant (26, 63) pour le guidage dans la direction axiale pour des volets de recouvrement (19, 20) de la cassette (6).
